# EUROPEAN PATENT SPECIFICATION

(11) **EP 1 563 852 B1**
(45) Date of publication and mention of the grant of the patent: **21.11.2007**
(21) Application number: 02785448.8
(22) Date of filing: 18.11.2002
(51) Int. Cl.: A61K 51/04, C07D 215/28, C07D 215/30, A61P 25/28

(54) **COMPOUNDS WHICH CAN BE USED TO DIAGNOSE AND MONITOR DISEASES ASSOCIATED WITH THE FORMATION OF AMYLOID PROTEIN FIBRILS**
VERBINDUNGEN ZUR DIAGNOSE UND ÜBERWACHUNG VON ERKRANKUNGEN IN ZUSAMMENHANG MIT DER BILDUNG VON AMYLOIDFIBRILLEN
COMPOSES UTILES POUR LE DIAGNOSTIC ET LE SUIVI DE MALADIES ASSOCIEES A LA FORMATION DE FIBRILLES PROTEIQUES AMYLOIDES

(43) Date of publication of application: 17.08.2005
(73) Proprietor: Cetir Centre Medic, S.A., 08029 Barcelona (ES); Catalana de Dispensacion, S.A., 08950 Esplugues de Llobregat (ES); Barnatron, S.A., 08950 Esplugues de Llobregat (ES)
(72) Inventor: SETOAIN QUINQUER, Jorge, E-08034 Barcelona (ES); PIERA PENA, Carlos, E-08190 Sant Cugat (ES); RAMIREZ DE ARELLANO SERNA, Isabel, E-08022 Barcelona (ES)
(74) Representative: ABG Patentes, S.L.
(86) International application number: PCT/ES2002/000537
(87) International publication number: WO 2004/045651

(56) References cited:
- WO-A-02/24652
- WO-A-98/06403
- US-A- 4 360 509
- US-A- 2002 025 944
- DATABASE CAPLUS [Online] Retrieved from STN Database accession no. 1999:472241 & JP 11 204 260 A 30 July 1999
- DATABASE CAPLUS [Online] ANDO ET AL.: 'Rapid synthesis of I-131 and I-125 labeled chinoform' Retrieved from STN Database accession no. 1975:31226 & RADIOISOTOPES vol. 23, 1974, pages 6 - 9

## Description

### Field of the Invention

The present invention refers to compounds which can be used to diagnose and monitor diseases associated with the formation of amyloid protein fibrils, to their use in the diagnosis of said diseases, to pharmaceutical compositions containing said compounds, and finally, to methods of preparing said compounds.

### Background of the Invention

It is known that diseases such as Alzheimer, Parkinson's, Huntington and Creutzfeld-Jacob, cystic fibrosis, late onset diabetes and motor neuron disease, amongst others, occur with the formation of amyloid protein fibrils from precursor proteins. These fibrils may present themselves in systemic form or as localised insoluble deposits. It is currently accepted that fibril forming amyloid proteins are characterised by being positive for Congo Red, being fibrillar by electron microscopy, and having a crossed beta structure by X-ray diffraction. More than 20 amyloid protein precursor proteins are known, some examples being light and heavy chain immunoglobulins, transthyretin, apolipoprotein Al, gelsolin, lysozyme, the Aβ precursor protein, prion proteins, the atrial natriuretic factor, prolactin and insulin.

Abnormal accumulations of two types of amyloid protein deposits in brain regions involved in learning tasks and memory, such as the hippocampus, are observed specifically in patients with Alzheimer's disease after post-mortem examination. Of these two types, the extracellular amyloid plaques are the most characteristic of Alzheimer's disease, while the intracellular neurofibrillary tangles may also be found in other types of neurodegenerative diseases.

The amyloid plaque is made up of dystrophic neurites and other altered astrocytes, as well as microglia which envelopes an insoluble fibrillar core. These fibrils are made up of a series of proteins which are generically called β-amyloid proteins, two of which, Aβ40 and Aβ42 are predominant. It has also been demonstrated that certain transition metals are intrinsic of the composition of β-amyloid aggregates, given that β-amyloid proteins have the ability to bond metals through specific locations for Cu and Zn. This bond is the one that mediates the precipitation of these proteins and, therefore, high concentrations of metals such as Cu and Zn are found in the neocortex and even higher concentrations in the β-amyloid plaques of Alzheimer patients. β-amyloid proteins are generated from corresponding precursor proteins which are expressed in a ubiquitous manner on cell surfaces. It is accepted today, although there is no irrefutable proof thereof, that the formation and accumulation of β-amyloid proteins, be it in prefibrillar or diffusible form or as plaque itself, is a starting and necessary factor in Alzheimer pathogenesis, which, in turn, precedes neurodegeneration. There is also a consensus in that therapeutic strategy design must be geared towards these processes.

In daily medical practice, neuropsychological examination and clinical observation of symptoms such as cognitive decline and systematic elimination of other possible causes of said symptoms is the standard and universally recognised method for determining whether a patient has probabilities of being affected by Alzheimer's disease or of already being in a first symptomatic stage (minimum symptoms of invalidity). The only way today of confirming these diagnoses with complete certainty is post-mortem brain examination. In these examinations, synapse counting is the most precise marker. However, the amount of amyloid plaque deposited is approximately correlated with the severity of the symptoms at the time of death of the patient. More specifically, it has been observed that the increase in the total amount of β-amyloid protein 1-42 in the hippocampus and in some cortical regions correlates perfectly with the early onset of clinical symptoms and their subsequent progression.

In this context, the possibility of obtaining functional images of the amount and distribution of amyloid plaques could serve as an important complement to the diagnosis of presymptomatic stages of the disease. Furthermore, the development of new *in vivo* brain amyloid deposit neuroimaging techniques could be much more transcendental for the evaluation of therapeutic alternatives. More specifically, given the need to test therapeutic agents that may inhibit the production of or redissolve β-amyloid proteins and thus reduce or prevent development of the disease it is crucial, for example, to be able to make informed decisions on matters such as: what type of patients are admitted in clinical tests, when the treatment is applied and how the progress of the treatment is evaluated.

In a chronic and non-infectious disease such as Alzheimer's disease, it is logical to assume that the first clinical trials of new substances potentially able to modify the course of the disease must be therapeutic rather than preventive. The results of these first trials have already been presented at the 8^{th} International Conference on Alzheimer's Disease (Stockholm, 20-25 July 2002) and a summary thereof has been published in Science magazine (New Alzheimer's treatments that may ease the mind, Science, 297, 1260-1262). It is urgent, therefore, for these and other efforts not to be in vain, to develop sensitive and precise methods that allow to assess the progress or regression of the disease during pharmacological treatment in a manner as objective and as quantitative as possible.

Amongst the imaging diagnostic methods available for this type of monitoring the Non-Invasive Radiotracer Imaging (NIRI) techniques are very suitable, as they allow visualising and quantifying specific events at molecular level such as those involved here. Of these techniques, there are two methods that provide high resolution images, one that uses positron emitting isotopes such as ¹⁸F and ¹¹C (Positron Emission Tomography: PET) and one that uses simple photon emitting radionuclides such as ^{99m}Tc and ¹²³I that allow obtaining planar images or images by Single Photon Emission Computerised tomography (SPECT).

Two research strategies, among others, are currently directed towards achieving a method that allows assessing the progress of Alzheimer's disease during a pharmacological treatment, but none of these have yet corroborated their strategy in systems *in vivo.* One of these consists in the intravenous administration of radioiodinated (¹²⁵I) β-amyloid protein which, when passing through the blood-brain barrier (BBB) may incorporate itself onto the neuritic plaques of the brain and thus allow its locating. This approach presents several problems such as, for example, that proteins are susceptible to degradation, that they have problems going through the blood-brain barrier, that they penetrate tissue with difficulty and that they are difficult to produce in high quantities. In this field, the most important efforts have been geared towards improving the permeability of β-amyloid proteins with regard to the BBB. One of the first strategies has consisted in joining the β-amyloid protein to protein vectors for which specific transporters exist in the BBB (Saito Y., et al. ; Vector-mediated delivery of 125I-labeled β-amyloid peptide Aβ1-40 through the blood-brain barrier and binding to Alzheimer's disease amyloid of the Aβ1-40/vector complex; Proc. Natl. Acad. Sci. U.S.A. 92 (1995) 10227-10231*).* In more recent approaches these vectors are the natural polyamines spermine and putrescine. It has been observed that the radioiodinated β-amyloid peptide (1-40) with putrescine conjugates bond better than the non-conjugated form and almost exclusively to the dense amyloid deposits of neuritic plaques in rats. This bonding does not take place on the innumerable diffuse deposits of β-amyloid protein that are not surrounded by dystrophic neurites and which, as would be desirable, could be indicators of precocious lesions (Wengenack T.M. et al.; "Targeting amyloid plaques in vivo"; Nat. Biotechnol. 18 (2000) 868-872)*.*

The other main line of research proposes the use of colorants with *in vitro* affinity for amyloid plaques as radiopharmaceutical tracers. One of the first jobs with Chrysamine G, an amyloid colorant which penetrates the BBB, has demonstrated the viability of this approach by staining amyloid plaques with technetium in different brain regions. Other researchers have tried to redesign, colorants such as Congo Red in order to accommodate in its molecule radioisotopes such as ^{99m}Tc, which have been demonstrated to be useful to visualise amyloid lesions *in vitro* (Zhen W.; "Synthesis and amyloid binding properties of rhenium complexes: preliminary progress toward a reagent for SPECT imaging of Alzheimer's disease brain" J. Med. Chem. 42 (1999) 2805-2815*).* One of the last attempts has been performed based on a new fluorescent colorant called BSB [(*trans, trans),*-1-bromo-2,5-bis-(3-hydroxycarbonyl-4-hydroxy)styrylbenzene]. BSB is capable of bonding to amyloid plaques in transgenic mice but it also has affinity for other β-sheet rich protein deposits such as intraneuronal neurofibrils and other bodies such as Lewy bodies which are common to other neurodegenerative diseases such as Parkinson's (Skovronsky D.M. et al.; In vivo detection of amyloid plaques in a mouse model of Alzheimer's disease; Proc. Natl. Acad. Sci. U.S.A. 97 (2000) 7609-7614*).*

On the other hand, US patent US 4 360 509 discloses the use of radioactive indium and 8-hydroxyquinoline chelates to locate inflammatory reactions in hot blooded animals by means of non-invasive imaging techniques. International patent application WO 00 76966 discloses rhodamine derivatives labelled with radioisotopes which are used for the diagnosis of diseases related to the amyloid protein deposition.

There is, therefore, a need for the identification and preparation of new alternative compounds that are useful for diagnosis and monitoring of diseases related to protein deposition in the central nervous system and which, furthermore, do not present the disadvantages of the compounds already used in the state of the art.

### Summary of the Invention

After exhaustive and laborious research, the applicant has prepared some compounds which, due to their structure are capable of interacting with the amyloid protein fibrils, including those which appear as amyloid plaques and affect the central nervous system, and which are surprisingly also capable of going through the blood-brain barrier. Said compounds are, therefore, useful for the diagnosis and monitoring of diseases associated with the formation of amyloid protein fibrils and, furthermore, do not present the drawbacks of the compounds and methods used up to now in the state of the art, such as the degradation of said compounds, their low permeability with respect to the blood-brain barrier, their low specificity, etc. With these compounds it is therefore possible to effect a diagnosis or a monitoring of the aforementioned diseases in animals, transgenic animals and, particularly, in humans.

One aspect of the present invention consists in the use of the compounds of General Formula I 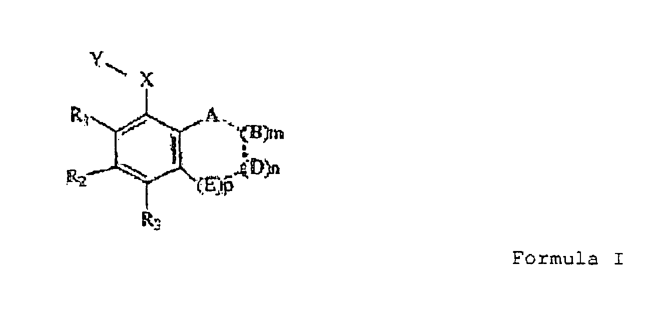 wherein:
X represents O, S;
Y represents H or, along with X, where X = O, a carbohydrate radical;
A represents N or NR₄;
B represents CR₅, NR₅ or N;
D represents CR₆, NR₆ or N;
E represents CR₇, NR₇ or N;
with the condition that the ring containing group A has a maximum of two nitrogen atoms in its structure;
m, n and p represent: 0 or 1, where m + n + p = 2 or 3;
the dashed lines - - - - represent a single or double bond;
R₁, R₂, R₃, R₄, R₅, R₆ and R₇ each independently represent a radioactive isotope, H, a halogen or a radical optionally having a radioactive isotope, said radical being chosen from: C₁-C₆ alkyl, OH, C₁-C₆ polyhydroxyalkyl, C₁-C₆ alkoxyl, C₁-C₆ alkoxyalkyl, (CH₂)q-OR', wherein q is 1, 2 or 3, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R'', (CH₂)ᵣ-CO-R', wherein r is 1, 2 or 3 and Rph, wherein Rph represents a non substituted or substituted phenol group, the possible substituents of the phenol group being any of the meanings of R₃-R₇ except for a phenol group;
R' is H or a C₁₋₃ alkyl group;
R'' is H, a C₁-C₆ alkyl group or a C₁-C₆ alkyloxy group;
with the condition that only one of R₁, R₂, R₃, R₄, R₅, R₆, R₇, X and Y is or has a radioactive isotope; in the preparation of a composition for diagnosis and/or monitoring of diseases associated with the formation of amyloid protein fibrils, particularly those that appear as amyloid plaques and affect the central nervous system.

A second aspect of the present invention consists in the use of the compounds of General Formula II. 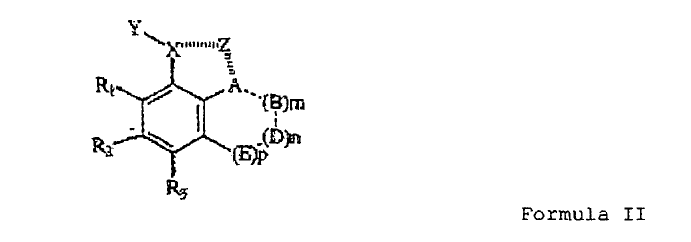 wherein:
X represents O, S;
Y represents H or, along with X, where X = O, a carbohydrate radical;
Z represents a metal or rare earth cation that may or may not be radioactive;
the l l l l l l l l line represents a coordinate bond;
A represents N or NR₄;
B represents CR₅, NR₅ or N;
D represents CR₆, NR₆ or N;
E represents CR₇, NR₇ or N;
with the condition that the ring containing substituent A has a maximum of two nitrogen atoms in its structure;
m, n and p represent: 0 or 1, where m + n + p = 2 or 3;
the dashed lines - - - - represent a single or double bond;
R₁, R₂, R₃, R₄, R₅, R₆ and R₇ each independently represent a radioactive isotope, H, a halogen or a radical optionally having a radioactive isotope, said radical being chosen from: C₁-C₆ alkyl, OH, C₁-C₆ polyhydroxyalkyl, C₁-C₆ alkoxyl, C₁-C₆ alkoxyalkyl, (CH₂)_{q}-OR', wherein q is 1, 2 or 3, CF₃, CH₂-CH₂F, O-CH₂-CH₂F, CH₂-CH₂-CH₂F, CN, NO₂, 0(CO)R', OR' , 5R', COOR' -SO₃H, (CH₂)r-CO₂R", (CH₂)r-CO-R', wherein r is 1, 2 or 3 and Rph, wherein Rph represents a non substituted or substituted phenol group, the possible substituents of the phenol group being any of the meanings of R₁-R₇ except for a phenol group;
R' is H or a C₁₋₃ alkyl group;
R" is H, a C₁-C₆ alkyl group or a C₁-C₆ alkyloxy group;
with the condition that only one of R₁, R₂, R₃, R₄, R₅, R₆, R₇, X, Y or Z is or has a radioactive isotope; in the preparation of a composition for diagnosis and/or monitoring of diseases associated with the formation of amyloid protein fibrils, particularly those that appear as amyloid plaques and affect the central nervous system.

A third aspect of the invention consists in the use of the compounds of General Formula III. 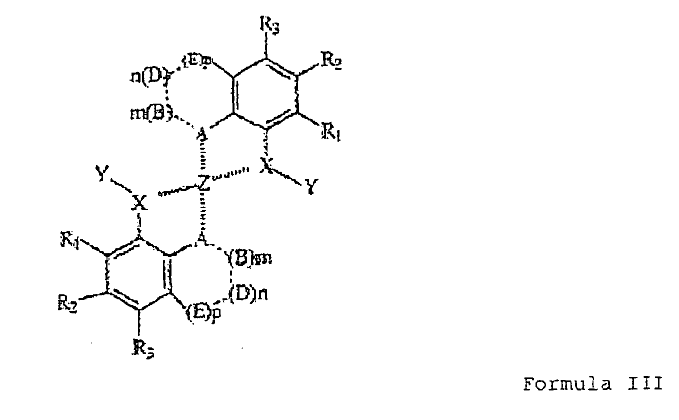 wherein:
X represents O, S;
Y represents H or, along with X, where X = O, a carbohydrate radical;
Z represents a metal or rare earth cation that may or may not be radioactive;
the |||||||| line represents a coordinate bond;
A represents N or NR₄;
B represents CR₅, NR₅ or N;
D represents CR₆, NR₆ or N;
E represents CR₇, NR₇ or N;
with the condition that the ring containing substituent A has a maximum of two nitrogen atoms in its structure;
m, n and p represent: 0 or 1, where m + n + p = 2 or 3;
the dashed lines - - - - represent a single or double bond; R₁, R₂, R₃, R₄, R₅, R₆ and R₇ each independently represent a radioactive isotope, H, a halogen or a radical optionally having a radioactive isotope, said radical being chosen from: C₁-C₆ alkyl, OH, C₁-C₆ polyhydroxyalkyl, C₁-C₆ alkoxyl, C₁-C₆ alkoxyalkyl, (CH₂)q-OR', wherein q is 1, 2 or 3, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R'', (CH₂)r-CO-R', wherein r is 1, 2 or 3 and Rph, wherein Rph represents a non substituted or substituted phenol group, the possible substituents of the phenol group being any of the meanings of R₁-R₇ except for a phenol group;
R' is H or a C₁₋₃ alkyl group;
R" is H, a C₁-C₆ alkyl group or a C₁-C₆ alkyloxy group;
with the condition that only one of R₁, R₂, R₃, R₄, R₅, R₆, R₇, X, Y or Z is or has a radioactive isotope; in the preparation of a composition for diagnosis and/or monitoring of diseases associated with the formation of amyloid protein fibrils, particularly those that appear as amyloid plaques and affect the central nervous system.

Within the context of the present invention, diseases associated with the formation of amyloid protein fibrils, particularly those that appear as amyloid plaques and affect the central nervous system, are understood to be diseases such as Alzheimer's, Parkinson's, Huntington, Creutzfeld-Jacob, cystic fibrosis, late onset diabetes, motor neuron disease, Mediterranean fever, Muckle-Wells syndrome, idiopathic myeloma, amyloid polyneuropathy, amyloid cardiomyopathy, senile systemic amyloidosis, hereditary cerebral haemorrhage with amyloidosis, Down syndrome, Creutzfeld-Jacob disease, Kuru, Gerstmann-Straussler-Schienker syndrome, thyroid medullar carcinoma, amyloid valve deposits, amyloidosis in dialysis patients, inclusion body myositis, amyloid muscular deposits, Sickle Cell Parkinson anaemia, type 2 diabetes, amongst others.

A fourth aspect of the invention refers to compounds having the following structures:
a) Compounds of General Formula I 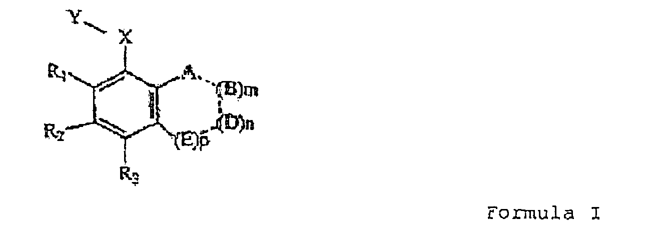 wherein:
   X represents O, S;
   Y represents H or, along with X, where X = O, a carbohydrate radical;
   A represents N or NR₄;
   B represents CR₅, NR₅ or N;
   D represents CR₆, NR₆ or N;
   E represents CR₇, NR₇ or N;
   with the condition that the ring containing group A has a maximum of two nitrogen atoms in its structure;
   m, n and p represent: 0 or 1, where m + n + p = 2 or 3;
   the dashed lines - - - - represent a single or double bond;
   R₁, R₂, R₃, R₄, R₅, R₆ and R₇ each independently represent a radioactive isotope, H, a halogen or a radical optionally having a radioactive isotope, said radical being chosen from: C₁-C₆ alkyl, OH, C₁-C₆ polyhydroxyalkyl, C₁-C₆ alkoxyl, C₁-C₆ alkoxyalkyl, (CH₂)q-OR', wherein q is 1, 2 or 3, CF₃, CH₂-CH₂F, O-CH₂-CH₂F, CH₂-CH₂-CH₂F, CN, NO₂, O(CO)R', OR', SR', COOR' -SO₃H, (CH₂) r-CO₂R", (CH₂)r-CO-R', wherein r is 1, 2 or 3 and Rph, wherein Rph represents a non substituted or substituted phenol group, the possible substituents of the phenol group being any of the meanings of R₁-R₇ except for a phenol group;
   R' is H or a C₁₋₃ alkyl group;
   R" is H, a C₁-C₆ alkyl group or a C₁-C₆ alkyloxy group;
   with the condition that R₁, R₂, R₃, R₄, R₅, R₆, R₇, X and Y are not all simultaneously H, and
   with the condition that only one of R₁, R₂, R₃, R₄, R₅, R₆, R₇, X and Y is or has a radioactive isotope;
   Within the Formula I compounds, those satisfying the ratio m + n + p= 3 are preferred.
b) Compounds of General Formula II 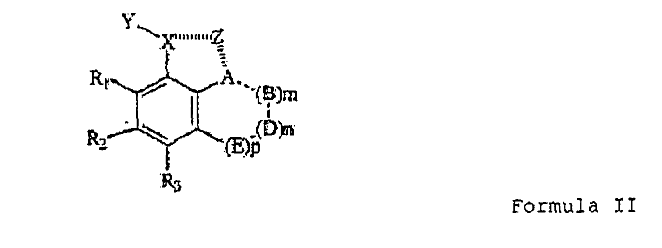 wherein:
   X represents O, S;
   Y represents H or, along with X, where X = O, a carbohydrate radical;
   Z represents a metal or rare earth cation that may or may not be radioactive ;
   the | | | | | | | | line represents a coordinate bond;
   A represents N or NR₄ ;
   B represents CR₅, NR₅ or N;
   D represents CR₆, NR₆ or N;
   E represents CR₇, NR₇ or N;
   with the condition that the ring containing group A has a maximum of two nitrogen atoms in its structure;
   m, n and p represent: 0 or 1, where m + n + p = 2 or 3;
   the dashed lines - - - - represent a single or double bond;
   R₁, R₂, R₃, R₄, R₅, R₆ and R₇ each independently represent a radioactive isotope, H, a halogen or a radical optionally having a radioactive isotope, said radical being chosen from: C₁-C₆ alkyl, OH, C₁-C₆ polyhydroxyalkyl, C₁-C₆, alkoxyl, C₁-C₆ alkoxyalkyl, (CH₂)q-OR', wherein q is 1, 2 or 3, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R'', (CH₂)r-CO-R', wherein r is 1, 2 or 3 and Rph, wherein Rph represents a non substituted or substituted phenol group, the possible substituents of the phenol group being any of the meanings of R₁-R₇ except for a phenol group;
   R' is H or a C₁₋₃, alkyl group;
   R" is H, a C₁-C₆ alkyl group or a C₁-C₆, alkyloxy group;
   with the condition that R₁, R₂, R₃, R₄, R₅, R₆, R₇, X and Y are not all simultaneously H, and
   with the condition that only one of R₁, R₂, R₃, R₄, R₅, R₆, R₇, X, Y or Z is or has a radioactive isotope;
   and, finally,
c) Compounds of General Formula III 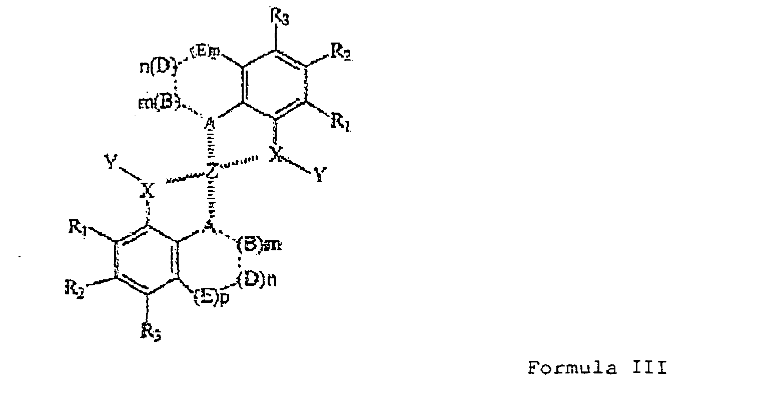 wherein:
   X represents O, S;
   Y represents H or, along with X, where X = O, a carbohydrate radical;
   Z represents a metal or rare earth cation that may or may not be radioactive;
   the |||||||| line represents a coordinate bond;
   A represents N or NR₄;
   B represents CR₅, NR₅ or N;
   D represents CR₆, NR₆ or N;
   E represents CR₇, NR₇ or N ;
   with the condition that the ring containing group A has a maximum of two nitrogen atoms in its structure;
   m, n and p represent: 0 or 1, where m + n + p = 2 or 3;
   the dashed lines - - - - represent a single or double bond;
   R₁, R₂, R₃, R₄, R₅, R₆ and R₇ each independently represent a radioactive isotope, H, a halogen or a radical optionally having a radioactive isotope, said radical being chosen from: C₁-C₆ alkyl, OH, C₁-C₆ polyhydroxyalkyl, C₁-C₆ alkoxyl, C₁-C₆ alkoxyalkyl, (CH₂)q-OR', wherein q is 1, 2 or 3, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R", (CH₂)r-CO-R', wherein r is 1, 2 or 3 and Rph, wherein Rph represents a non substituted or substituted phenol group, the possible substituents of the phenol group being any of the meanings of R₁-R₇ except for a phenol group;
   R' is H or a C₁₋₃ alkyl group;
   R" is H, a C₁-C₆ alkyl group or a C₁-C₅ alkyloxy group;
   with the condition that R₁, R₂, R₃, R₁, R₅, R₆, R₇, X and Y are not all simultaneously H, and
   with the condition that only one of R₁, R₂, R₃, R₄, R₅, R₆, R₇, X, Y or Z is or has a radioactive isotope;
   and with the condition that when
   A is N,
   B, D and E are all CH,
   X is O, and
   m, n and p are all 1,
   then R₁, R₂ and R₃ are not all H.

Preferred compounds for use in the diagnosis and monitoring of diseases associated with the formation of amyloid protein fibrils are chosen from the following:
a) Compounds according to General Formula I:
   Analogues with radioactive iodine.
      5-chloro-7-[¹³³I]iodo-8-hydroxyquinoline
      5-chloro-7-[¹²¹I]iodo-8-hydroxyquinoline
      5-[¹²³I]iodo-7-iodo-8-hydroxyquinoline
      5-iodo-7-[¹²³I]iodo-8-hydroxyquinoline
      5-[¹²⁴I]iodo-7-iodo-8-hydroxyquinoline
      5-iodo-7-[¹²¹I]iodo-8-hydroxyquinoline
   Analogues with radioactive fluorine.
      5-chloro-7-[¹⁸F]fluoro-8-hydxoxyquinoline
      5-[¹⁸F]fluoro-7-iodo-8-hydroxyquinoline
   Analogues with radioactive carbon.
      5-chloro-7-iodo-S-[¹¹C]methoxyquinoline
   Glucuronides.
      5-chloro-7-[¹²³] iodo-8-hydroxyquinoline glucuronide
      5-chloro-7-[¹²¹I] iodo-8-hydroxyquinoline glucuronide
      5-chloro-7-[¹⁸F] fluoro-8-hydroxyquinoline glucuronide
      5-[¹⁸F]fluoro-7-iodo-8-hydroxyquinoline glucuronide
      5-chloro-7-iodo-8-[¹¹C]methoxyquinoline glucuronide
   Analogues with a single halogen atom.
      5- [¹²³I] -8-hydroxyquinoline
      5-[¹²⁴I]-8-hydroxyquinoline
      7-[¹²³I]-8-hydroxyquinoline
      7- [¹²⁴I]-8-hydroxyquinoline
      5-[¹⁸F]-8-hydroxyquinoline
      5-[¹⁸F]-8-hydroxyquinoline
b) Compounds according to General Formula II:
   Metal complexes with 5-chloro-7-[²²³I]iodo-8-hydroxyquinoline
      5-chloro-7-[¹²³I]iodo-8-hydroxyquinoline Fe (II) complex
      5-chloro-7-[¹²³I]iodo-8-hydroxyquinoline Cu(II) complex
      5-chloro-7-[¹²³I]iodo-8-hydroxyquinoline Zn(II) complex
      5-chloro-7-[¹²³I]iodo-8-hydroxyquinoline Mn (II) complex
   Metal complexes with 5-chloro-7-[¹²⁴I]iodo-8-hydroxyquinoline
      5-chloro-7-[¹²⁶I]iodo-8-hydroxyquinoline Fe(II) complex
      5-chloro-7-[¹²⁴I]iodo-8-hydroxyquinoline Cu (II) complex
      5-chloro-7-[¹²⁴I]iodo-8-hydroxyquinoline Zn(II) complex
      5-chloro-7-[¹²⁴I]iodo-8-hydroxyquinoline Mn(II) complex
   Metal complexes with 5-chloro-7-[¹⁸F]fluoro-8-hydroxyquinoline
      5-chloro-7-[¹⁹F]fluoro-8-hydroxyquinoline Fe(II) complex
      5-chloro-7-[¹⁸F]fluoro-8-hydroxyquinoline Cu(II) complex
      5-chloro-7-[¹⁸F]fluoro-8-hydroxyquinoline Zn(II) complex
      5-chloro-7-[¹⁸F]fluoro-8-hydroxyquinoline Mn(II) complex
   Metal complexes with 5-[¹⁸F]fluoro-7-iodo-8-hydroxyquinoline
      5-[¹⁸F]fluoro-7-iodo-8-hydroxyquinoline Fe (II) complex
      5-[¹⁸F]fluoro-7-iodo-8-hydroxyquinoline Cu(II) complex
      5-[¹⁸F]fluoro-7-iodo-8-hydroxyquinoline Zn(II) complex
      5-[¹⁸F] fluoro-7-iodo-8-hydroxyquinoline Mn(II) complex
   Metal complexes with 5-chloro-7-iodo-8-[¹¹C] methoxyquinoline
      5-Chloro-7-iodo-8-[¹¹C]methoxyquinoline Fe (II) complex
      5-chloro-7-iodo-8-[¹¹C]methoxyquinoline Cu(II) complex
      5-chloro-7-iodo-8-[¹¹C]methoxyquinoline Zn(II) complex
      5-chloro-7-iodo-8-[¹¹C]methoxyquinoline Mn(II) complex
   Metal complexes with 5-chloro-7-iodo-8-hydroxyquinoline
      5-chloro-7-iodo-8-hydroxyquinoline ^{99m}Tc complex
      5-chloro-7-iodo-8-hydroxyquinoline ¹¹¹In complex
      5-chloro-7-iodo-8-hydroxyquinoline ^{2C1}Tl complex
      5-chloro-7-iodo-8-hydroxyquinoline ⁶⁷Ga complex
      5-chloro-7-iodo-8-hydroxyquinoline ⁶⁸Ga complex
      5-chloro-7-iodo-8-hydroxyquinoline ⁶⁷Cu complex
      5-chloro-7-iodo-8-hydroxyquinoline ⁶⁴Cu complex
c) Compounds according to General Formula III:
   Bis-chelate metal complexes with 5-chloro-7-[¹²³I]iodo-8-hydroxyquinoline
      5-chloro-7-[¹²³I]iodo-8-hydroxyquinoline Fe(II) bis-chelate complex
      5-chloro-7-[¹²³I]iodo-8-hydroxyquinoline Cu(II) bis-chelate complex
      5-chloro-7-[¹²³I]iodo-8-hydroxyquinoline Zn(II) bis-chelate complex
      5-chloro-7-[¹²³I]iodo-8-hydroxyquinoline Mn(II) bis-chelate complex
   Bis-chelate metal complexes with 5-chloro-7-[¹²⁴I]iodo-8-hydroxyquinoline
      5-chloro-7- [¹²⁴I] iodo-8-hydroxyquinoline Fe (II) bis-chelate complex
      5-chloro-7-[¹²⁴I]iodo-8-hydroxyquinoline Cu(II) bis-chelate complex
      5-chloro-7- [¹²⁴I]iodo-8-hydroxyquinoline Zn (II) bis-chelate complex
      5-chloro-7-[¹²⁴I]iodo-8-hydroxyquinoline Mn(II) bis-chelate complex
   Bis-chelate metal complexes with 5-chloro-7-[¹⁸F]fluoro-8-hydroxyquinoline
      5-chloro-7- [¹⁸F]fluoro-8-hydroxyquinoline Fe(II) bis-chelate complex
      5-chloro-7-[¹⁸F]fluoro-8-hydroxyquinoline Cu(II) bis-chelate complex
      5-chloro-7-[¹⁸F]fluoro-6-hydroxyquinoline Zn(II) bis-chelate complex
      5-chloro-7-[¹⁸F]fluoro-8-hydroxyquinoline Mn(II) bis-chelate complex
   Bis-chelate metal complexes with 5-[¹⁸F] fluoro-7-iodo-8-hydroxyquinoline
      5-[¹⁸F]Fluoro-7-iodo-8-hydroxyquinoline Fe(II) bis-chelate complex
      5-[¹⁸F] fluoro-7-iodo-8-hydroxyquinoline Cu(II) bis-chelate complex
      5-[¹⁸F]fluoro-7-iodo-8-hydroxyquinoline Zn(II) bis-chelate complex
      5-[¹⁸F]fluoro-7-iodo-8-hydroxyquinoline Mn(II) bis-chelate complex
   Bis-chelate metal complexes with 5-chloro-7-iodo-8-[¹¹C]methoxyquinoline
      5-chloro-7-iodo-8-[¹¹C]methoxyquinoline Fe(II) bis-chelate complex
      5-chloro-7-iodo-8-[¹¹C]methoxyquinoline Cu(II) bis-chelate complex
      5-chloro-7-iodo-8-[¹¹C]methoxyquinoline Zn(II) bis-chelate complex
      5-chloro-7-iodo-8-[¹¹C]methoxyquinoline Mn(II) bis-chelate complex
   Bis-chelate metal complexes with 5-chloro-7-iodo-8-hydroxyquinoline
      5-chloro-7-iodo-8-hydroxyquinoline ^{99m}Tc bis-chelate complex
      5-chloro-7-iodo-8-hydroxyquinoline ¹¹¹In bis-chelate complex
      5-chloro-7-iodo-8-hydroxyquinoline ²⁰¹Tl bis-chelate complex
      5-chloro-7-iodo-8-hydroxyquinoline ⁶⁷Ga bis-chelate complex
      5-chloro-7-iodo-8-hydroxyquinoline ⁶⁸Ga bis-chelate complex
      5-chloro-7-iodo-8-hydroxyquinoline ⁶⁷Cu bis-chelate complex
      5-chloro-7-iodo-8-hydroxyquinoline ⁶⁴Cu bis-chelate complex

The compounds of the present invention are prepared according to methods known in the state of the art. Thus:

The compounds of Formula I are prepared according to a method comprising making a quinoline derivative react:
a) with an electrophilic aromatic halogenation reagent incorporating a radioactive halogen atom, or;
b) with a radioactive halogenated derivative to effect an aromatic nucleophilic substitution reaction.

The compounds of Formula II are prepared according to a method comprising making a quinoline derivative react:
a) with a metal or rare earth cation, or,
b) with a radioactive isotope of these elements,
such that the metal or rare earth cation or the radioactive isotope of these elements is in a suitable oxidation state so as to produce the corresponding chelating product of Formula II.

The compounds of Formula III are prepared according to conventional methods known in the state of the art. The preferred methods comprise making a quinoline derivative react with:
c) a metal or rare earth cation, or,
d) a radioactive isotope of these elements in a suitable oxidation state so as to produce the corresponding chelating product defined in Formula III.

The present invention is also directed to a method of imaging a tissue of the central nervous system of a human, transgenic animal or animal, using a compound of formulae I, II and III as defined above.

According to a preferred embodiment, the tissue is affected by diseases associated with the formation of amyloid protein fibrils, particularly those that appear as amyloid plaques.

### Preparative Examples

### EXAMPLE 1:

### Preparation of 5-chloro-8-hydroxy-7-[¹²⁵I]iodoquinoline by isotope exchange.

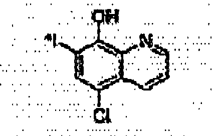

A 1-3 mCi solution of Na¹²⁵I is introduced in a 10 mL balloon and is evaporated to dryness at 100°C under a nitrogen current. The corresponding 7-iodoquinoline is added (100 mg) dissolved in 2 mL of a suitable solvent. The balloon is joined to a reflux condenser and the bath temperature is raised. The reaction mixture is stirred under nitrogen atmosphere for a certain time and is let to cool. Water is added and the product that is collected by filtration is rinsed well with water. It is recrystallised and the purity is determined by means of thin layer radiochromatography.

### EXAMPLE 2:

### Preparation of 5-chloro-8-hydroxy-7-trimethylstannylquinoline.

Hexamethylditin (1.31 mmol) is added to an 0.88 mmol mixture of iodine derivative (5-chloro-8-hydroxy-7-iodoquinoline) and tetrakis-triphenylphosphine palladium(0) (0.05 g) in 12 mL of 1,4-dioxane and the mixture is heated under reflux under nitrogen atmosphere for 6.5 hours. After cooling, the crude reaction product is filtered and the insoluble material is washed with ethyl acetate. The solvent is eliminated under reduced pressure, obtaining a yellowish oil which is subject to silica gel column chromatography to produce a solid with a 65% yield.

### EXAMPLE 3:

### Synthesis of 5-chloro-8-hydroxy-7-[¹²³I]iodoquinoline from the organotin derivative using chloramine T.

[¹²³I]sodium iodide (2-20 mCi) is added to a solution of the trimethyltin derivative described in Example 2 (200-300 ug) in 300 uL of ethanol, followed by chloramine T (100 ug) in 1N HCl (100 uL). After stirring for 5 minutes the reaction is stopped with an aqueous solution of metabisulphite (50 mg/mL, 100 uL) and is injected in the RP-HPLC. The radioiodinated derivative fraction obtained is collected with a 98% radiochemical purity.

### EXAMPLE 4:

### Synthesis of 5-chloro-8-hydroxy-7-[¹³¹I]idoquinoline from the organotin derivative using hydrogen peroxide.

The radioiodinated derivative is obtained from the tributyltin derivative prepared in an analogous manner to the organotin derivative described in example 2 by suspension in methanol and treatment with Na¹³¹I and hydrogen peroxide at room temperature, as described below. The reaction takes place by adding 50 uL of H₂O₂ (3% w/v) to a mixture formed by 50 uL of the tributyltin derivative (100 ug/50 uL EtOH), 1.5 mCi of [¹²⁵I] sodium iodide (specific activity 2200 Ci/mmol) and 100 uL of 1N HCl in a sealed vial. The reaction is -stirred at room temperature for 10 minutes and is finalised after adding 100 uL of a saturated solution of anhydrous Na₂SO₃ and the solvent is evaporated by means of a nitrogen current. It is neutralized with sodium bicarbonate and extracted with ethyl acetate. The extract is dried by passing the solution over a column of anhydrous Na₂SO₄ and the solvent is evaporated by means of nitrogen current. The crude reaction product is purified by HPLC obtaining a radiochemical purity over 85%. The fractions are dried and the residue is extracted again with EtOAc. The different EtOAc extracts are concentrated under nitrogen atmosphere until dry. The residue is dissolved in EtOH. The radiochemical purity of the final product exceeds 98% (by HPLC).

### EXAMPLE 5:

### Synthesis of 5-chloro-8-hydroxy-7-[¹³¹I] iodoquinoline using chloramine T.

1.0 mL of an 0.02 M KH₂PO₄ buffer solution (pH 4.8) is added to a vial containing 5-chloro-8-hydroxyquinoline. The mixture is heated to 40°C and is stirred to obtain a transparent solution which is cooled to room temperature. 10 mL of an 0.1 N NaOH solution containing 10.0 mCi of [¹³¹I]sodium iodide is added and the vial is closed with a Teflon stopper. A 7.5 ug chloramine T (N-chloro-p-toluenesulphonamide sodium salt) solution in 30 uL of the 0.02 M KH₂PO₄ buffer solution is added. It is mechanically stirred at room temperature for 5 minutes and then manually for some seconds. After continuing stirring for 5 more minutes an aqueous solution of NaHSO₃ (1.4 mg/mL) is added and the reaction mixture is analysed by thin layer chromatography. The solution is passed over an anionic exchange column to eliminate free radioiodine.

### EXAMPLE 6:

### Synthesis of 5-chloro-8-hydroxy-7-[¹²⁵I]iodoquinoline using chloramine T.

A 30 nmol solution of 5-chloro-8-hydroxyquinoline in 30 uL of ethanol is added to 10 mCi of [¹²⁵I] sodium iodide (4.5 ug, 330 Ci/mmol, 30 nmol) in 0.001 N NaOH (30 uL). A solution of chloramine T (13 ug, 50 nmol) in water (10 uL) is added. The mixture is heated to 60°C during 45 minutes. 100 uL of 0.1 N NH₄Cl are added and it is extracted with ether. The reaction result is analysed by thin layer chromatography (Merck F254 Silica gel) in the appropriate eluent and then treated as in the previous example.

### EXAMPLE 7:

### Synthesis of 5-chloro-8-hydroxy-8-hydroxy-7-[¹³¹I]iodoquinoline using Iodogen^{™}.

100 uL of a Iodogen solution (1,3,4,6-tetrachloro-3-alpha,6-alpha-diphenylglycoluril) (1.0 mg/mL in CH₂Cl₂) are added to a 5 mL vial. Dichloromethane evaporates from the vial due to nitrogen current. A 5-chloro-8-hydroxyquinoline solution (0.5 mg/0.5 mL) in 0.02M KH₂PO₄, pH 4.8 is added to the vial, which is then closed with a Teflon stopper and a solution containing approximately 10 mCi of [¹³¹I]sodium iodide is introduced by means of a syringe. The mixture is stirred at room temperature for 30 minutes. A radiochemical purity exceeding 99% is observed by thin layer chromatography in silica gel using suitable eluents.

### EXAMPLE 8:

### Preparation of 5-chloro-8-hydroxy-7-[¹²⁵I]iodoquinoline using Iodo-beads.

The Iodo-beads are previously washed with a 0.1 M sodium phosphate buffer solution at pH = 6.5. The necessary amount of Iodo-beads is added to an Na¹²⁵I solution in buffer solution at a concentration of 1 mCi for each 100 ug of product to be iodinated. Between 5 and 500 ug of the phenol derivative dissolved in the same buffer solution are then added thereto. It is left to react between 2 and 15 minutes at room temperature. The reaction is stopped by separating the Iodo-beads from the solution by decantation. The Iodo-beads are washed with the buffer solution in order to recover all the radioiodinated derivative.

### EXAMPLE 9:

### Synthesis of 5-chloro-8-hydroxy-7-[¹²²I]iodoquinoline using peracetic acid.

0.19 umol of 5-chloro-8-hydroxyquinoline precursor dissolved in 50 uL of ethanol are mixed in a vial with a known volume of a buffered solution at pH 2 which is twice the volume of the basic commercial [¹²³I]iodine solution. This solution is introduced in the vial containing the radioactive iodine, followed by 50 uL of a 3.2% solution of peracetic acid (obtained from a stock solution at 32% w/v). The sealed vial is heated to 6.5°C for 12 minutes. It is left to cool and an aqueous solution of NaHSO₃ is added. The radiochemical yield is purified and determined by chromatographic methods such as those described in previous examples.

### EXAMPLE 10:

### Preparation of 5-chloro-7-[¹⁸F]fluoro-hydroxyquinoline by direct radiofluorination.

Direct radiofluorination of 100 umol of the phenol derivative 5-chloro-8-hydroxyquinoline is performed by dissolving the compound in a mixture of trifluoroacetic acid and glacial acetic acid (1:1) through which freshly prepared acetyl hypofluorite ([¹⁸F]CH₃-COOF) is bubbled. The solvent is evaporated and the residue is purified by means of HPLC.

### EXAMPLE 11:

### Synthesis of 5-[¹⁸F]fluoro-hydroxyquinoline from 5-fluoro-8-hydroxyquinoline by isotope exchange.

The radioactive fluoride [¹⁸F] is transferred to a 5 mL borosilicate reaction vial and is azeotropically dried with acetonitrile in the presence of 4.0 mg of K₂CO₃ and 14.6 mg of Kryptofix 2.2.2®. The precursor fluoride derivative dissolved in 0.5 mL of DMSO is added to the vial containing the dry radioactive fluoride, the K₂CO₃ and the Kryptofix 2.2.2® and the isotope exchange reaction is performed heating to 110, 130 and 160°C for 0-30 minutes to optimise the reaction. Purification of the radiofluorinated derivative is performed by reverse phase HPLC. The corresponding fractions are collected and concentrated under reduced pressure.

### EXAMPLE 12:

### Preparation of 5-chloro-8-hydroxy-7-[¹²³I]iodoguinoline from 5-chloro-7-fluoro-8-hydroxyquinoline.

The starting material dissolved in 600 uL of ethanol is added to 100 uL of a solution A (containing 200 umol of SnSO₄, 2 mmol of gentisic acid, 2 mmol of monohydrate citric acid, 10 ul of glacial acetic acid dissolved in 10% acetic acid), 25 uL of a solution B (containing 400 umol of CuSO₄.5H₂O dissolved in 10 mL of water) and 65 uL of glacial acetic acid. The vial is placed in an ultrasound bath for 15 minutes, and 10 uL of Na¹²³I (about 1 mCi) are added. N₂ is passed through the mixture and it is heated to 60°C for 1 hour. It is left to cool at room temperature and the crude reaction product is purified by means of HPLC.

### EXAMPLE 13 :

### Preparation of 5-[¹⁸F] fluoro-hydroxyquinoline by aromatic nucleophilic substitution.

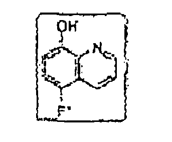

The corresponding halo- or nitro- derivative is made to react with the fluorine-18 ion by aromatic nucleophilic substitution using the [¹⁸F] FK-K222 complex in DMSO and
heating to 150-180°C in the conventional manner for 10 minutes or activating the reaction mixture with a 100 Watt microwave during 1-2.5 minutes. The characterisation and isolation of the radiolabelled product is performed as in Example 11.

### EXAMPLE 14:

### Synthesis of 5-chloro-7-[¹⁹F]fluoro-8-hydroxyquinoline from the deprotected precursor.

The electrophilic radiofluorination agent [¹⁹F]CH₃COOF, prepared from 100 umol [¹⁸F]F₂, or [¹⁸F]OF₂ (100 umol), is made to bubble for 10 minutes at room temperature in a solution (101 umol) of the trimethyltin derivative 5-chloro-8-hydroxy-7-trimethylstannyl-quinoline or 5-chloro-8-hydroxy-7-tributylstannyl-quinoline in 10 mL of Freon-11 (CFCl₃). The solvent is evaporated at 50°C with a nitrogen current and the residue is dissolved in 10 mL of methylene chloride and is transferred to a chromatographic column packed with Na₂S₂O₃ (2.5 cm) and silica gel (9.5 cm) which has previously been equilibrated with ether. It is eluted with ether. The solvent is evaporated and the solution is purified by means of semi-preparative HPLC.

### EXAMPLE 15:

### Synthesis of 5-chloro-7-[¹⁸F]fluoro-8-hydroxyquinoline from the protected precursor.

The electrophilic radio fluorination agent [¹⁸F]CH₃COOF, prepared from 100 umol [¹⁸F]F₂, or [¹⁸F]OF₂ (100 umol), is made to bubble for 10 minutes at room temperature in a solution (101 umol) of the trimethyltin derivative 5-chloro-8-t-butoxycarbonyloxy-7-trimethylstannyl-quinoline or 5-chloro-8-t-butoxycarbonyloxy-7-tributylstanny-quinoline in 10 mL of Freon-11 (CFCl₃). The solvent is evaporated at 50°C with a nitrogen current and the residue is dissolved in 10 mL of methylene chloride and is transferred to a chromatographic column packed with Na₂S₂O₃ (2.5 cm) and silica gel (9.5 cm) which has previously been equilibrated with ether. It is eluted with ether. The solvent is evaporated and the derivative is hydrolysed in the.presence of a 48% hydrobromic acid solution at 130°C for 10 min. After cooling, the reaction mixture is partially neutralised with a 3 N NaOH solution and purified by means of HPLC.

### EXAMPLE 16:

### Preparation of 5-chloro-7-[¹⁸F]fluoro-8-hydroxyquinoline by substituting trimethylammonium with fluorine.

260 mg (1.5 equiv.) of HNMe₂.HCl and 430 mg (1.5 equiv.) of K₂CO₃ are added to a 2.1 mmol solution of the precursor fluorine derivative in a mixture of 30 mL of DMSO and 10 mL of water at 10°C. After stirring at 10°C for 10 minutes, the solution is heated under reflux for 24 hours and left to cool, diluted with water (50 mL) and the product is extracted with ether. After processing the reaction, the residue is purified by silica gel chromatography. In a second reaction step the following process is followed: 1.45 equiv. of methyl trifluoromethanesulphonate are added to a solution of 0.65 mmol of dimethyl amino derivative in 2 mL of toluene. The solution is stirred for 1 hour at room temperature under nitrogen atmosphere and is then diluted with 100 mL of water. The product is extracted with dichloromethane, the solvent is evaporated and the remaining product is ground with diethyl ether. In a final stage, about 2.0-3.5 mg (6.5-11.3 umol) of the methyl trifluoromethanesulphonate derivative are dissolved in 600 uL of freshly distilled DMSO and added directly to the tube containing the K[¹⁸F]F-K222 anhydrous complex. The tube is heated on a heating block at 145-150°C without stirring for 2 minutes or is placed in a 100 W microwave oven for 1 minute. The reaction mixture is left to cool and is diluted with 3 mL of water and filtered through a C18 Sep Pack cartridge. The cartridge is washed with 3.0 mL of water and partially dried for 0.5 minutes applying nitrogen current. The radiofluorinated derivative is eluted from the cartridge with DCM (3 mL) followed by two more washings each of 1 mL.

### EXAMPLE 17:

### Synthesis of the clioquinol glycoconjugate, 5-chloro-8-yl-iodoquinoline-beta-D-glucuronic acid sodium salt.

A mixture of 5-chloro-8-hydroxy-7-iodoquinoline (50 mg, 0.164 mmol), methyl 1-bromo-1-deoxy-2,3,4-tri-O-acetyl-D-glucopyranoside uronate (65 mg, 0.164 mmol), CaSO₄.H₂O (35 mg) in pyridine (1.5 mL) is stirred at room temperature for a few minutes. Ag₂CO₃ (35 mg) is added to the reaction and the suspension is stirred at room temperature for 20 hours, protected from light. Once the reaction has finished and after isolating the product, the deprotection of the hydroxyl protecting groups is performed using a 1 N NaOH solution. The reaction is diluted with dichloromethane, filtered and the solvent is removed at reduced pressure. The glycoconjugate is purified by means of flash column chromatography. (TLC: CH₂Cl₂/MeOH 99/1, eluent: CH₂Cl₂/MeOH 99.5/0.5). NMR (400 MHz, CDCl3) 2.04 (s, 3H, Ac), 2.09 (s, 3H, Ac), 2.13 (s, 3H, Ac), 3.68 (s, 3H, Me), 3.99 (d, 1H, 5'H), 5.40-5.52 (m, 3H, 2',-3', -4' -H), 6.29 (d, 1H, 1'-H), 7.56 (m, 1H, 3H), 7.99 (s, 1H, 6-H), 8.52 (d, 1H, 4-H), 8.93 (s, 1H, 2-H).

### EXAMPLE 18:

### Radioiodination of the clioquinol glucuronide with Iodogen^{™}:

### Synthesis of the 5-chloro-8-hydroxy-7-[¹³¹I]iodoquinoline glucuronide.

10 mg of Iodogen^{™} are dissolved in 2 mL of dichloromethane in a tube containing small crystals in order to increase contact of the solution with the Iodogen^{™} (1,3,4,6-tetrachloro-3α, 6α-diphenylglycoluril). The solvent is evaporated and a white film is observed inside the tube and on the small crystals. 1 mL of an aqueous solution of the corresponding glucuronide (4 mg/mL) is added and then 7.4x10⁷ Bq (2 mCi) of Na¹³¹I is added. The solution is kept at room temperature while stirring for 10 minutes. The reaction is followed by TLC and the Rf of the radiochromarography-labelled product is determined. It can be observed that a single radioiodinated product has been obtained with a radioactive yield between 90 and 95%.

### EXAMPLE 19:

### Radioiodination of the clioquinol glucuronide with chloramine T: Synthesis of the 5-chloro-8-hydroxy-7-[¹²⁵I] iodoquinoline glucuronide.

A 4 mg/mL solution of chloramine T in 50 mM of disodium phosphate at pH 7 is prepared. 25 uL of a standard solution of the glycoconjugate in 50 uM of sodium phosphate at pH 7 is added to 2 uL of a 0.5 mCi/uL Na¹²⁵I solution. The vial is closed and 25 uL of the chloramine T solution are added, this mixture is stirred for 30 seconds. Once the reaction has finished 100 uL of 12.6 mM sodium metabisulphite are added. It is stirred for 10 seconds. The reaction product is purified by filtration on gel using a Sephadex G-25 or G-50 column.

### EXAMPLE 20:

### Radioiodination of the clioguinol glucuronide with Iodo-beads: Synthesis of the 5-chloro-8-hydroxy-7-[¹²⁶I]iodoquinoline glucuronide.

25 uL of a solution of the glycoconjugate in 50 uM of sodium phosphate at pH 7 are added to 2 uL of a 0.5 mCi/uL Na¹²⁵I solution. The vial is closed and some Iodo-beads are added and it is stirred for 30 seconds. Once the reaction is finished the polymer is separated from the supernatant. 100 uL of 12.6 mM sodium metabisulphite are added to this solution. It is stirred for 10 seconds. The reaction product is purified by filtration on gel using a Sephadex G-25 or G-50 column.

### EXAMPLE 21:

### Synthesis of the 5,7-dichloro-8-hydroxyquinoline [¹¹¹In]indium complex.

The trihydrate [¹¹¹In] indium chloride (¹¹¹InCl₃.3H₂O), 1.37 g, 5.0 mmol) and the 5,7-dichloro-8-hydroxy-quinoline (2.14 g, 10 mmol) are dissolved in ethanol (200 mL) and the solution is concentrated to dryness at reduced pressure. The yellowish crude product obtained is recrystallised from ethanol after adding water to give the 5,7-dichloro-8-hydroxyquinoline [¹¹¹In] indium complex.

### EXAMPLE 22:

### Synthesis of the 8-hydroxyquinoline [⁶⁷Ga]gallium chelator.

The [⁶⁷Ga] gallium chelator is prepared by adding [⁶⁷Ga] gallium trichloride (⁶⁷GaCl₃) dissolved in an 0.05 M HCl solution to a 7 mM aqueous solution of 8-hydroxyquinoline at pH 3.5. After about 25 minutes of stirring the pH increases to 6. The extraction of the product with chloroform leads to the chelator, which is obtained with a 90% yield.

### EXAMPLE 23:

### Preparation of the 8-hydroxyquinoline [^{99m}Tc]technetium chelator.

The 8-hydroxyquinoline (0.51 mmol) is dissolved in 3 mL of a 0.1 N NaOE solution and the solution pH is adjusted to pH = 3.5 with 1 N HCl. 0.3 mL of an SnCl₂ solution (20 mg, 0.11 mmol in 10 mL of 1 N HCl) are added and the pH is again adjusted with 0.1 N NaOH. It is stirred for 5 minutes and 80 uCi of technetium-99m are added in the form of sodium pertechnetate. The chelator is obtained by extracting with chloroform with a yield exceeding 90%.

### EXAMPLE 24:

### Synthesis of the 8-hydroxyquinoline [⁶⁴Cu]copper chelator.

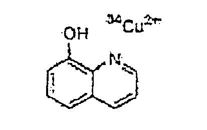

A [⁶⁴Cu] copper (II) chloride (⁶⁴CuCl₂) solution is diluted 10 times with a 0.1 M ammonium acetate buffer solution of pH 5.5. The [⁶⁴Cu] copper acetate is added to 8-hydroxyquinoline and the final volume is adjusted to 1.0-1.5 mL with the buffer solution. After incubating at room temperature for 45 minutes, the extraction of the ⁶⁴Cu derivative product with chloroform leads to the chelator, which is obtained with a 90% yield.

### EXAMPLE 25:

### Synthesis of the 8-hydraxyquinoline [⁶⁷Cu] copper chelator.

An aliquot of the starting solution (⁶⁷Cu²¹ /HCl) is evaporated to dryness under nitrogen current and reconstituted with an 0.25 N acetate buffered solution (pH = 5.5). The ligand (0.2 mg) is dissolved in DMSO (1 mg/10 uL) and is added to 50 uL of the [⁶⁷Cu] copper acetate solution (50 uL). After adding 50 uL of ethanol, the [⁶⁷Cu] copper derivative is filtered through a polytetrafluoxoethylene (PFTE) membrane. The radiochemical purity is determined by thin layer chromatography in silica gel plaques eluting with ethanol.

### EXAMPLE 26:

### Synthesis of 5-chloro-8-hydroxy-7-[¹²⁵I]iodoquinoline copper chelators.

### METHOD A: Chelation of 5-chloro-8-hydroxy-7-[¹²⁵I] iodoquinoline.

A copper (II) chloride solution is diluted 10 times with 0.1 M ammonium acetate of pH 5.5. This solution is added to 5-chloro-8-hydroxy-7-[¹²⁵I]iodoquinoline and the final volume is adjusted to 1.0-1.5 mL with a 0.1 M ammonium acetate buffer solution at pH = 5.5. After incubating at room temperature for 45 minutes, the extraction of the solution with chloroform leads to the chelator, which is obtained with a 90% yield.

### METHOD B: Radioiodination of the 5-chloro-8-hydroxyquinoline copper chelator.

A 30 nmol solution of 5-chloro-8-hydroxyquinoline copper chelator in 30 uL of ethanol is added to 10 mCi of [¹²⁵I]sodium iodide (4.5 ug, 330 Ci/mmol, 30 nmol) in 0.001 N NaOH (30 uL). A chloramine T solution (13 ug, 50 nmol) in water (10 uL) is added. The mixture is heated to 60°C for 45 minutes. 100 uL of 0.1 N NH₄Cl is added and extracted with ether. The product is analysed by thin layer chromatography (Silica gel, Merck F254) in the appropriate eluent.

### EXAMPLE 27:

### Synthesis of 5-chloro-8-hydroxy-7-[¹²⁵I]iodoquinoline zinc chelators.

### METHOD A: Chelation of 5-chloro-8-hydroxy-7-[¹²⁵I] iodoquinoline.

Zn(OAc)₂.2H₂O (60 mmol) is added to a suspension of 5-chloro-8-hydroxy-7-[¹²⁵I]iodoquinoline derivative (60 mmol) in methanol and the mixture is stirred for 19 hours at room temperature. The product is filtered and washed with methanol followed by petroleum ether and it is left to dry in the dryer.

### METHOD B: Radioiodination of the 5-chloro-8-hydroxyquinoline zinc chelator.

A solution of 5-chloro-8-hydroxyquinoline zinc chelator in 30 uL of ethanol is added to 10 mCi of [¹²⁵I] sodium iodide (4.5 ug, 330 Ci/mmol, 30 nmol) in 0.001 N NaOH (30 uL). A solution of chloramine T (13 ug, 50 nmol) in water (10 uL) is added. The mixture is heated to 60°C for 45 minutes. 100 uL of 0.1 N NH₄Cl are added and extracted with ether. The reaction product is analysed by thin layer chromatography (Silica gel, Merck F254) in the appropriate eluent.

### EXAMPLE 28:

### Radioiodination of the 5-chloro-8-hydroxyguinoline manganese (II) chelator.

A solution of 5-chloro-8-hydroxyquinoline manganese (II) chelator in 30 uL of ethanol is added to 10 mCi of [¹²⁵I]sodium iodide (4.5 ug, 330 Ci/mmol, 30 nmol) in 0.001 N NaOH (30 uL). A solution of chloramine T (13 ug, 50 nmol) in water (10 uL) is then added. The mixture is heated to 60°C for 45 minutes. After adding 100 uL of 0.1 N NH₁Cl it is extracted with ether. The obtained product is analysed by thin layer chromatography (Silica gel, Merck F254) in the appropriate eluent.

### EXAMPLE 29:

### Radioiodination of the 5-chloro-8-hydroxyquinoline iron (II) chelator.

A solution of 5-chloro-8-hydroxyquinoline iron (II) chelator in 30 uL of ethanol is added to 10 mCi of [¹²⁵I]sodium iodide (4.5 ug, 330 Ci/mmol, 30 nmol) in 0.001 N NaOH (30 uL). After adding a solution of chloramine T (13 ug, 50 nmol) in water (10 uL), the mixture is heated to 60°C for 45 minutes. The reaction is processed by adding 100 uL of 0.1 N NH₄Cl and extracting with ether. The reaction monitoring is performed by thin layer chromatography (Silica gel, Merck F254) in the appropriate eluent.

### Examples of Activity as a Biological Marker

### EXAMPLE 30:

### Quantitative autoradiography in transgenic mice and control mice.

The 5-chloro-8-hydroxy-7-[¹²³I]iodoquinoline radioactive derivative is injected in transgenic mice (Tg2576) and control mice via the jugular vein under anaesthesia using a 30 g syringe. A series of controls and transgenic mice are examined. After 2, 4, 6 hours of the intravenous injection, the brains of each mouse are removed, they are quickly cooled with dry ice and dissected using a Mokron HM 505E cryostat apparatus, placing them on glass sheets and drying them at room temperature. For the film autoradiography the sheets are placed in a MICROM optical densitometer obtaining greyscale images of the distribution of the radiopharmaceutical in the histological sections. Measured optical density is expressed as a percentage (%) of the maximum. Images were obtained with an optimal detection of amyloid plaques of the mice brains.

### EXAMPLE 31:

### MicroPET images of transgenic mice

All the animal experiments were performed according to the established animal protocol. The transgenic mice (Tg2576) were anaesthetised via intravenous injection with a 40 uL solution of ketamine and xylazine (4:1) before the injection of the tracer radiolabelled with ¹⁸F: 5-[¹⁸F]-fluoro-8-hydroxy-7-iodoquinoline. The mice were placed in supine position and scanned using microPET. A dynamic microPET study was performed using 15 planes and 15 to 16 frames (dynamic sequence: 6 x 30 s, 4 x 1 min, 2 x 5 min, 2 x 10 min, and 1-2 x 20 min) for a total acquisition time of 55-77 min. Images were reconstructed using the appropriate algorithms (Filter-back projection algorithm) and a cut-off frequency of 0.5. Images were reconstructed by means of a filtered iteration resulting in an image resolution of 1.8 mm and a volumetric resolution of about 6 mm³. Transverse planes were reoriented to obtain the coronal and axial sections of the mice brains. Regional uptake is expressed in activity per gram of tissue. The images showed an adequate detection of the amyloid plaques in mice brains.

### EXAMPLE 32:

### SPECT images of transgenic mice

The study was performed using transgenic mice (Tg2S76) in groups of 5 animals for each time value. The tracers (with SPECT-detectable radionuclides) (1-5 uCi) dissolved in 100 uL of PBS or in the appropriate solvent were injected via the tail vein.

SPECT images were obtained after 4.5 hours from the administration of the 5-chloro-8-hydroxy-7- [¹²³I] iodoquinoline tracer by means of a double-head camera (MULTISPECT II, Siemens Medical Systems) equipped with parallel low energy high resolution collimators, using an energy window centred on the photopeak energy of the corresponding radionuclide (159KeV for ¹²³I).

⁵⁷Co sources were used which were placed on the head of the animal in order to aid reconstruction by means of anatomical references. 120 projections were obtained in a 360 degree turn, acquired in a 64 x 64 matrix. Two simultaneous acquisitions are performed, one using a window of 20% of the energy centred at 159 KeV for ¹²³I and another of 4% of the energy centred at 122 KeV for the ⁵⁷Co labels. Total exploration time was about 40 minutes. The SPECT images were reconstructed using a filter-back projection algorithm. The attenuation correction was performed using the Chang algorithm.

The semiquantitative analysis of the uptake was performed comparing the average/pixel counts in the region of interest with the average/pixel counts in control regions, The results were compared with those obtained in the macroautoradiographies. The images showed the distribution of the amyloid plaques in the mice brains.

## Claims

1. The use of compounds of General Formula I 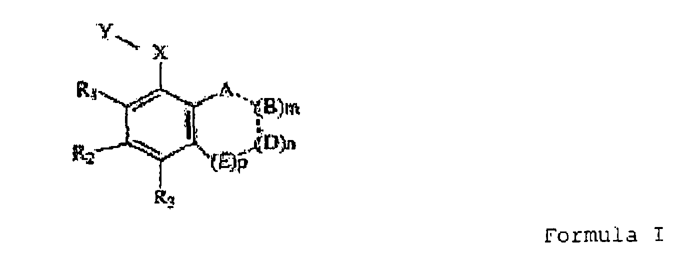 wherein:
X represents O, S;
Y represents H or, along with X, where X = O, a carbohydrate radical;
A represents N or NR₄;
B represents CR₅, NR₅ or N;
D represents CR₆, NR₆ or N;
E represents CR₇, NR₇ or N;
with the condition that the ring containing group A has a maximum of two nitrogen atoms in its structure;
m, n and p represent: 0 or 1, where m + n + p - 2 or 3;
the dashed lines - - - - represent a single or double bond ;
R₁, R₂, R₃, R₄, R₅, R₆ and R₇ each independently represent a radioactive isotope, H, a halogen or a radical optionally having a radioactive isotope, said radical being chosen from: C₁-C₆ alkyl, OH, C₁-C₆ polyhydroxyalkyl, C₁-C₆ alkoxyl, C₁-C₆, alkoxyalkyl, (CH₇)q-OR', wherein q is 1, 2 or 3, CF₃, CH₂-CH₂F, O- CH₂-CH₂F, CH₂-CH₂-CH₂F, CN, NO₂, O(CO)R', OR', SR', COOR' -SO₃H, (CH₂)r-CO₂R'', (CH₂)r-CO-R', wherein n r is 1, 2 or 3 and Rph, wherein Rph represents a non substituted or substituted phenol group, the possible substituents of the phenol 1 group being any of the meanings of R₁-R₇ except for a phenol group;
R' is H or a C₁₋₃ alkyl group ;
R" is H, a C₁-C₆ alkyl group or a C₁-C₆ alkyloxy group;
with the condition that only one of R₁, R₂, R₃, R₄, R₅, R₆, R₇, X and Y is or has a radioactive isotope;
in the preparation of a composition for diagnosis and/or monitoring of diseases associated with the formation of amyloid protein fibrils, particularly those that appear as amyloid plaques and affect the central nervous system.

2. The use of compounds of General Formula II. 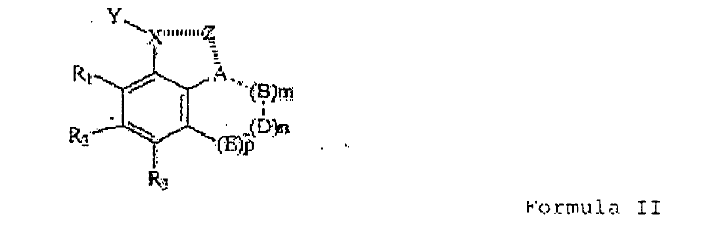 wherein :
X represents O, S ;
Y represents II or, along with X, where X = O, a carbohydrate radical;
Z represents a metal or rare earth cation that may or may not be radioactive ;
the |||||||| line represents a coordinate bond;
A represents N or NR₄;
B represents CR₅, NR₅, or N;
D represents CR₆, NR₆ or N;
E represents CR₇, NR₇, or N;
with the condition that the ring containing substituent A has a maximum of two nitrogen atoms in its structure;
m, n and p represent: 0 or 1, where m + n + p = 2 or 3;
the dashed lines - - - - represent a single or double bond;
R₁, R₂, R₃, R₄, R₅, R₆ and R₇ each independently represent a radioactive isotope, II, a halogen or a radical optionally having a radioactive isotope, said radical being chosen from: C₁-C₆ alkyl, OH, C₁-C₆ polyhydroxyalkyl, C₁-C₆ alkoxyl, C₁-C₆ alkoxyalkyl, (CH₂)q-OR', wherein q is 1, 2 or 3, CF₃, CH₂-CH₂F, O-CH₂-CH₂F, CH₂CH₂-CH₂F, CN, NO₂, O(CO)R', OR' , SR' , COOR' -SO₃H, (CH₂)r-CO₂R'', (CH₂)r-CO-R', wherein r is 1, 2 or 3 and Rph, wherein Rph represents a non substituted or substituted phenol group, the possible substituents of the phenol group being any of the meanings of R₁-R₇ except for a phenol group;
R' is II or a C₁₋₃ alkyl group;
R'' is H, a C₁-C₆ alkyl group or a C₁-C₆ alkyloxy group;
with the condition that only one of R₁, R₂, R₃, R₄, R₅, R₆, R₇, X, Y or Z is or has a radioactive isotope;
in the preparation of a composition for diagnosis and/or monitoring of diseases associated with the formation of amyloid protein fibrils, particularly those that appear as amyloid plaques and affect the central nervous system.

3. The use of compounds of General Formula III. 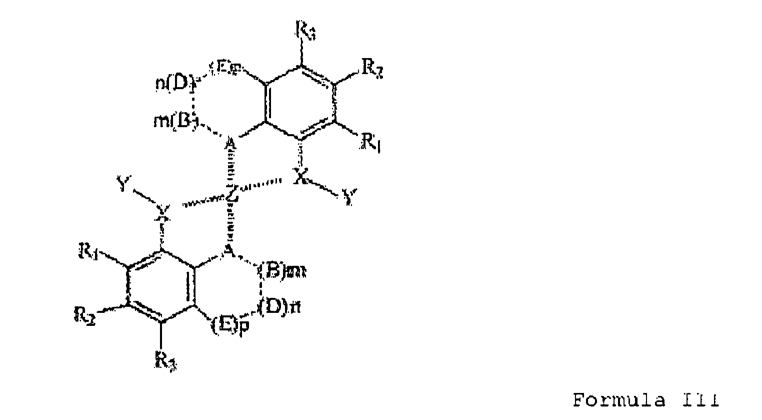 wherein:
X represents O, S;
Y represents H or, along with X, where X - O, a carbohydrate radical;
Z represents a metal or rare earth cation that may or may not be radioactive;
the l l l l l l l l line represents a coordinate bond;
A represents N or NR₄ ;
B represents CR₅, NR₅ or N;
D represents CR₆, NR₆ or N;
E represents CR₇, NR₇ or N;
with the condition that the ring containing substituent A has a maximum of two nitrogen atoms in its structure ;
in, n and p represent: 0 or 1, where m + n + p = 2 or 3;
the dashed lines - - - - represent a single or double bond;
R₁, R₂, R₃, R₄, R₅, R₆, and R₇ each independently represent a radioactive isotope, H, a halogen or a radical optionally having a radioactive isotope, said radical being chosen from : C₁-C₆ alkyl, OH, C₁ C₆ polyhydroxyalkyl, C₁-C₆ alkoxyl, C₁-C₆ alkoxyalkyl, (CH₂)q-OR', wherein q is 1, 2 or 3, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R'', (CH₂)r-CO-R', wherein r is 1, 2 or 3 and Rph, wherein Rph represents a non substituted or substituted phenol group, the possible substituents of the phenol group being any of the meanings of R₁-R₇ except for a phenol group;
R' is H or a C₁₋₃ alkyl group;
R'' is II, a C₁-C₆ alkyl group or a C₁-C₆ alkyloxy group; with the condition that only one of R₁, R₂, R₃, R₄, R₅, R₆, R₇, X, Y or Z is or has a radioactive isotope;
in the preparation of a composition for diagnosis and/or monitoring of diseases associated with the formation of amyloid protein fibrils, particularly those that appear as amyloid plaques and affect the central nervous system.

4. Use according to claims 1, 2 and 3 for diagnosis and/or monitoring in animals, transgenic animals, and particularly in humans, of diseases such as Alzheimer's, Parkinson's, Huntington, cystic fibrosis, late onset diabetes, motor neuron disease, Mediterranean fever, Muckle-Wells syndrome, idiopathic myeloma, amyloid polyneuropathy, amyloid cardiomyopathy, senile systemic amyloidosis, hereditary cerebral haemorrhage with amyloidosis, Down syndrome, Creutzfold-Jacob disease, Kuru, Gerstmann-Straussler-Schienker syndrome, thyroid medullar carcinoma, amyloid valve deposits, amyloidosis in dialysis patients, inclusion body myositis, amyloid muscular deposits, Sickle Cell Parkinson anaemia, type 2 diabetes, amongst others.

5. Compounds of General Formula I 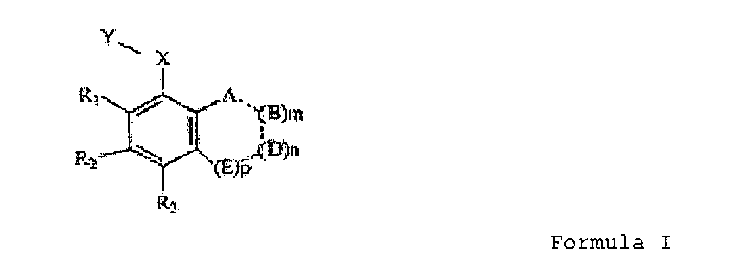 wherein:
X represents O, S;
Y represents H or, along with X, where X = O, a carbohydrate radical;
A represents N or NR₄;
B represents CR₅, NR₅, or N;
D represents CR₆, NR₆ or N;
E represents CR₇, NR₇ or N;
with the condition that the ring containing substituent A has a maximum of two nitrogen atoms in its structure;
m, n and p represent: 0 or 1, where m + n + p - 2 or 3;
the dashed lines - - - - represent a single or double bond;
R₁, R₂, R₃, R₄, R₅, R₆ and R₇ each independently represent a radioactive isotope, H, a halogen or a radical optionally having a radioactive isotope, said radical being chosen from: C₁-C₆ alkyl, OH, C₁-C₆ polyhydroxyalkyl, C₁-C₆ alkoxyl, C₁-C₆ alkoxyalkyl, (CH₂)q-OR', wherein q is 1, 2 or 3, CF₂, CH₂-CH₂F, O-CH₂-CH₂F, CH₂-CH₂-CH₂F, CN, NO₂, O(CO)R', OR', SR', COOR' -SO₃H, (CH₂) r-CO₂R", (CH₂)ᵣ-CO-R', wherein r is 1., 2 or 3 and Rph, wherein Rph represents a non substituted or subtituted phenol group, the possible substituents of the phenol group being any of the meanings of R₁-R₇ except for a phenol group;
R' is H or a C₁₋₃ alkyl group;
R'' is H, a C₁-C₆ alkyl group or a C₁-C₆ alkyloxy group;
with the condition that R₁, R₂, R₃, R₄, R₅, R₆, R₇, X and Y are not all simultaneously H, and
with the condition that only one of R₁, R₂, R₃, R₁, R₅, R₆, R₇, X and Y is or has a radioactive isotope;

6. Compounds of General Formula II 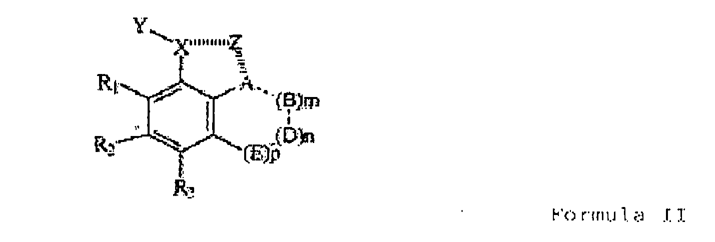 wherein:
X represents O, S;
Y represents H or, along with X, where X - O, a carbohydrate radical;
Z represents a metal or rare earth cation that may or may not be radioactive;
the IIIIIIII line represents a coordinate bond;
A represents N or NR₄;
B represents CR₅, NR₅ or N;
D represents CR₆, NR₆ or N;
E represents CR₇, NR₇ or N;
with the condition that the ring containing group A has a maximum of two nitrogen atoms in its structure ;
m, n and p represent.: 0 or 1, where m + n + p = 2 or 3 ;
the dashed lines - - - - represent a single or double bond;
R₁, R₂, R₃, R₄, R₅, R₆, and R₇ each independently represent a radioactive isotope, H, a halogen or a radical optionally having a radioactive isotope, said radical being chosen from: C₁-C₆, alkyl, OH, C₁ C₆ polyhydroxyalkyl, C₁-C₆ alkoxyl, C₁-C₆ alkoxyalkyl, (CH₂)q-OR', wherein q is 1, 2 or 3, CF₃, CH₂-CH₂F, O-CH₂-CH₂F, CH₂-CH₂-CH₂F, CN, NO₂, O(CO)R', OR', SR', COOR' -SO₃H, (CH₂)r CO₂R'', (CH₂)r-CO-R', wherein r is 1, 2 or 3 and Rph, wherein Rph represents a non substituted or substituted phenol group, the possible substituents of the phenol group being any of the meanings of R₁-R₇ except for a phenol group;
R' is H or a C₁₋₃ alkyl group;
R'' is H, a C₁-C₆ alkyl group or a C₁-C₆ alkyloxy group;
with the condition that R₁, R₂, R₃, R₄, R₅, R₆, R₇, X and Y are not all simultaneously H, and
with the condition that only one of R₁, R₂, R₃, R₄, R₅, R₆, R₇, X, Y or Z is or has a radioactive isotope;

7. Compounds of General Formula III 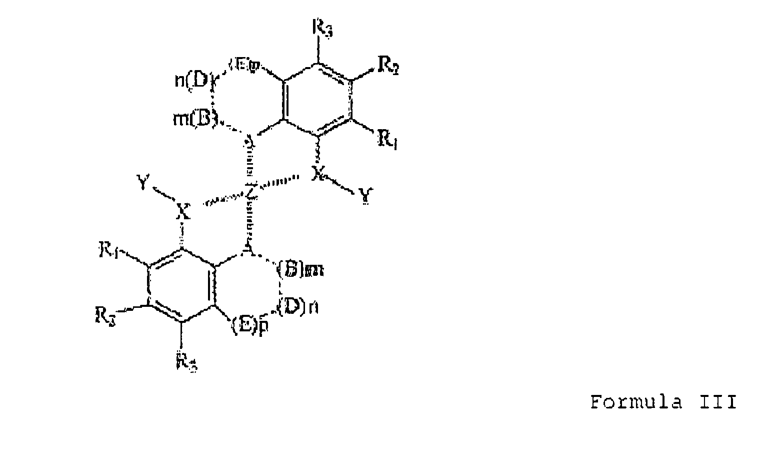 wherein:
X represents O, S;
Y represents H or, along with X, where X = O, a carbohydrate radical;
7, represents a metal or rare earth cation that may or may not be radioactive;
the l l l l l l l l line represents a coordinate bond;
A represents N or NR₄;
B represents CR₅, NR₅, or- N;
D represents CR₆, NR₆, or N;
E represents CR₇, NR₇ or N;
with the condition that the ring containing group A has a maximum or two nitrogen atoms in its structure;
m, n and p represent: 0 or 1, where m + n + p = 2 or 3 ;
the dashed lines - - - - represent a single or double bond;
R₁, R₂, R₃, R₄, R₅, R₆ and R₇ each independently represent a radioactive isotope, II, a halogen or a radical optionally having a radioactive isotope, said radical being chosen from: C₁ C₆ alkyl, OH, C₁-C₆ polyhydroxyalkyl, C₁-C₆ alkoxyl, C₁-C₆ alkoxyalkyl, (CH₂)_{q}-OR', wherein q is 1, 2 or 3, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R'', (CH₂)r-CO-R', wherein r is 1, 2 or 3 and Rph, wherein n Rph represents a non substituted or substituted phenol group, the possible substituents of the phenol group being any of the meanings of R₁-R₇ except for phenol group;
R' is H or a C₁₋₃ alkyl group;
R" is 11, a C₁-C₆ alkyl group or a C₁-C₆ alkyloxy group;
with the condition that only one of R₁, R₂, R₃, R₄, R₅, R₆, R₇, X, Y or Z is or has a radioactive isotope;
and with the condition that when
A is N,
B, D and E are all CH,
X is O, and
m, n and p are all 1,
then R₁, R₂ and R₃ are not all H

8. Compounds according to claim 5, **characterised by** being:
5-chloro-7*-* [¹²⁴I] iodo-8-hydroxyquinoline
5- [¹²⁴I] iodo-7-iodo-8-hydroxyquinoline
5-iodo-7- [¹²⁴I] iodo-8-hydroxyquinoline
5-chloro-7-[¹⁸F]Fluoro-8-hydroxyquinoline
5-[¹⁸F]fluoro-7-iodo-8-hydroxyquinoline
5-chloro-7-iodo-8- [¹¹C]methoxyquinoline
5-chloro-7-[¹²⁴I]iodo-8-hydroxyquinoline glucuronide
5-chloro-7-[¹²⁵I]iodo-8-hydroxyquinoline glucuronide
5-chloro-7-[¹³¹I]iodo-8-hydroxyquinoline glucuronide
5-chloro-7-[¹⁸F]fluoro 8-hydroxyquinoline glucuronide
5-[¹⁸F]fluoro-7- iodo-8-hydroxyquinoline glucuronide
5-chloro-7-iodo-8-[¹¹C]methoxyquinoline glucuronide glucuronide
5-[¹²⁴I]-8-hydroxyquinoline
7-[¹²⁴I]-8-hydroxyquinoline
5-[¹⁸F]-8-hydroxyquinoline

9. Compounds according to claim 5, **characterised by** being:
5-chloro-7-[¹²³I]iodo-8-hydroxyquinoline
5-iodo-7-[¹²³I]iodo-8-hydroxyquinoline
5-chloro-7-[¹²³I]iodo-8-hydroxyquinoline glucuronide
5-[¹²³I]-8-hydroxyquinoline
7-[¹²³I]-8-hydroxyquinoline

10. Compounds according to claim 6:
5-chloro-7- [¹²⁴I] iodo-8-hydroxyquinoline Fe (II) complex
5-chloro-7- [¹²⁴I] iodo-8 -hydroxyquinoline Cu(II) complex
5-chloro-7-[¹²⁴I] iodo- 8-hydroxyquinoline Zn (II) complex
5-chloro-7- [¹²⁴I] iodo-8-hydroxyquinoline Mn (II) complex
5-chloro-7-[¹²⁵I]iodo-8-hydroxyquinoline Fe (I) complex
5-chloro-7- [¹²⁵I] indo-8-hydroxyquinoline Cu (II) complex
5-chloro-7-[¹²⁵I] iodo-8-hydroxyquinoline Zn (II) complex
5-chloro-7-[¹²⁵I] iodo-8-hydroxyquinoline Mn (II) complex
5-chloro-7-[¹⁸F] fluoro-8-hydroxyquinoline Fe (II) complex
5-chloro-7-[¹⁸F] fluoro-8-hydroxyquinoline Cu (II) complex
5-chloro-7- [¹⁸F] fluoro-8-hydroxyquinoline Zn(II) complex
5-chloro-7- [¹⁸F] fluoro-8-hydroxyquinoline Mn (II) complex
5-[^{18F}]fluoro-7-iodo-8-hydroxyquinoline Fe (II) complex
5- [¹⁸F] fluoro-7-iodo-8-hydroxyquinoline Cu (II) complex
5-[¹⁸F] fluoro-7- iodo-8 -hydroxyquinoline Zn(II) complex
5- [¹⁸F] fluoro-7-iodo-8-hydroxyquinoline Mn(II) complex
5-chloro-7-iodo-8-[¹¹C]methoxyquinoline Fe (II) complex
5-chloro-7-iodo-8-[¹¹C]methoxyquinoline Cu (II) complex
5-chloro-7-iodo-8- [¹¹C]methoxyquinoline Zn(II) complex
5-chloro-7-iodo-8-[¹¹C]methoxyquinoline Mn(II) complex

11. Compounds according to claim 6:
5-chloro-7-[¹²³I] iodo-8-hydroxyquinoline Fe (II) complex
5-chloro-7- [¹²³I] iodo-8 -hydroxyquinoline Cu(II) complex
5-chloro-7-[¹²³I] iodo-8-hydroxyquinoline Zn (II) complex
5-chloro-7-[¹²³I] iodo-8-hydroxyquinoline Mn(II) complex

12. Compound According to claim 6 :
5-chloro-7-iodo-8-hydroxyquinoline ^{99m}Tc complex
5-chloro-7-iodo-8-hydroxyquinoline ¹¹¹In complex
5-chloro-7-iodo 8-hydroxyquinoline ²⁰¹Tl complex
5 chloro-7-iodo-8-hydroxyquinoline ⁶⁷Ga complex
5-chloro-7-iodo-8-hydroxyquinoline ⁶⁸Ga complex
5-chloro-7-iodo-8-hydroxyquinoline ⁶⁷Cu complex
5-chloro-7-iodo-8-hydroxyquinoline ⁶⁴Cu complex

13. Compounds according to claim 7:
5-chloro-7-[¹²⁴I]iodo-8-hydroxyquinoline Fe(II) bis-chelate complex
5-chloro-7-[¹²⁴I]iodo-8-hydroxyquinoline Cu(II) bis-chelate complex
5-chloro-7-[¹²⁴I]iodo-8-hydroxyquinoline Zn(II) bis-chelate complex
5 chloro-7-[¹²⁴I] iodo-8-hydroxyquinoline Mn (II) bis-cholate complex
5-chloro-7-[¹⁸F]fluoro-8-hydroxyquinoline Fe (II) bis-chelate complex
5-chloro-7-[¹⁸F]fluoro-8-hydroxyquinoline Cu (II) bis-chelate complex
5-chloro-7-[¹⁸F] fluoro-8-hydroxyquinoline Zn (II) bis-chelate complex
5-chloro-7-[¹⁸F]fluoro-8hydroxyquinoline Mn(II) bis-chelate complex
5-[¹⁸F] fluoro-7-iodo-8-hydroxyquinoline Fe (II) bis-chelate complex
5- [²⁸F] fluoro-7-iodo-8-hydroxyquinoline Cu(II) bis-chelate complex
5-[¹⁸F] fluoro-7-iodo-8-hydroxyquinoline Zn(II) bis-chelate complex
5-[¹⁸F] fluoro-7-iodo-8-hydroxyquinoline Mn(II) bis chelate complex
5-chloro-7-iodo-8-[¹¹C] methoxyquinoline Fe(II) bis chelate complex
5-chloro-7-iodo 8-[¹¹C]methoxyquinoline Cu(II) bis-chelate complex
5-chloro-7-iodo-8-[¹¹C]methoxyquinoline Zn(II) bis-chelate complex
5-chloro-7-iodo-8-[¹¹C]methoxyquinoline Mn(II) bis-chelate complex

14. Compounds according to claim 7:
5-chloro-7-[¹²³I]iodo-8-hydroxyquinoline Fe(II) bis-chelate complex
5-chloro-7-[¹²³I]iodo-8-hydroxyquinoline Cu(II) bis-chelate complex
5-chloro-7-[¹²³I]iodo-8-hydroxyquinoline Zn(II) bis-chelate complex
5-chloro-7-[¹²³I]iodo-8-hydroxyquinoline Mn(II) bis-chelate complex

15. Compound according to claim 7:
5-chloro-7-iodo-8-hydroxyquinoline ^{99m}Tc bis-chelate complex
5-chloru-7-iodo-8-hydroxyquinoline ¹¹¹In bis-chelate complex
5-chloro-7-Iodo-8-hydroxyquinoline ²⁰¹Tl bis-chelate complex
5-chloro-7-iodo-8-hydroxyquinoline ⁶⁷Ca bis-chelate complex
5-chloro-7-iodo-8-hydroxyquinoline ⁶⁸Ga bis-chelate complex
5-chloro-7-iodo-8-hydroxyquinoline ⁶⁷Cu bis-chelate complex
5-chloro-7-iodo-8-hydroxyquinoline ⁶⁴Cu bis-chelate complex

16. A pharmaceutical composition for diagnosis of diseases associated with protein deposition in the central nervous system comprising one of the compounds defined in claims 5 to 12.

17. A method for preparing the compounds defined in claims 5, 8 and 9 comprising:
a) making a quinoline derivative react with an electrophilic aromatic halogenation reagent incorporating a radioactive halogen atom, or
b) making a quinoline derivative react with a radioactive halogenated derivative to effect an aromatic nucleophilic substitution reaction.

18. A method tor preparing the compounds defined in claims 6, 10, 11 and 12 comprising:
a) making a quinoline derivative react with a metal or rare earth cation, or,
b) making a quinoline derivative react with a radioactive isotope of these elements
such that the metal or rare earth cation or the radioactive isotope of these elements is in a suitable oxidation state so as to produce the corresponding chelating product defined in claims 6, 10, 11 and 12.

19. A method for preparing the compounds defined in claim 7 comprising making a quinoline derivative react with:
a) a metal or rare earth cation, or,
b) a radioactive isotope or these elements.
in a suitable oxidation state so as to produce the corresponding chelating product defined in claims 7, 13, 14 and 15.

20. A method or imaging a tissue of the central nervous system of a human, transgenic animal or animal, using a compound or formulae 1, 11 and III as defined in claims 1, 2 and 3 respectively or a compound according to claims 8 to 15.

21. A method according to claim 20, wherein the tissue is attected by diseases associated with the formation of amyloid protein fibrils, particularly those that appear as amyloid plaques.

## Patentansprüche

1. Verwendung von Verbindungen der Allgemeinen Formel 1 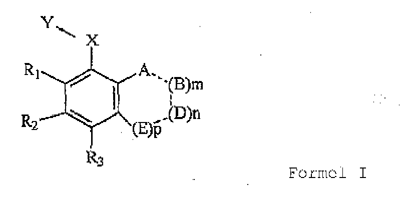 wobei:
X O, S darstellt ;
Y H oder zusammen mit X, wo X = O ist, ein Kohlenhydratrest darstellt;
A N oder NR₁, darstellt;
B CR₅, NR₅ oder N darstellt;
D CR₆, NR₆ oder N darstellt;
E CR₇, NR₇ oder N darstellt;
mit der Bedingung, dass der Ring, welcher die Gruppe A enthält, ein Maximum von zwei Stickstoffatomen in seiner Struktur aufweist;
m, n und p 0 oder 1 darstellen, wo m + n + p = 2 oder 3 ist;
die gestrichelten Linien - - - - eine Einfach- oder Doppelbindung darstellen;
R₁, R₂, R₃, R₄, R₅, R₆ und R₇ jeder unabhängig ein radioaktives Isotop, H, ein Halogen oder einen Rest optional mit einem radioaktiven Isotop darstellen, diesen Rest wählend aus: C₁-C₆ alkyl, OH, C₁-C₆ Polyhydroxylalkyl, C₁-C₆ Alkoxyl, C₁-C₆ Alkoxylalkyl, (CH₂)q-OR' , wobei q 1, 2 oder 3 ist, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R", (CH₂)r-CO-R', wobei r 1, 2 oder 3 ist, und Rph, wobei Rph eine unsubstuierte oder substituierte Phenolgruppe darstellt, wobei die möglichen Substituenten der Phenol gruppe eine jede der Bedeutungen von R₁-R₇ mit Ausnahme einer Phenolgruppe sind;
R' II oder eine C₁₋₃ Alkylgruppe ist;
R" H, eine C₁-C₆ Alkylgruppe oder eine C₁-C₆ Alkoxylgruppe ist;
mit der Bedingung, dass nur einer von R₁, R₂, R₃, R₄, R₅, R₆, R₇, X und Y ein radioaktives Isotop ist oder aufweist;
in der Erstellung einer Zusammensetzung zur Diagnose und /oder Überwachung von Krankheiten, welche mit der Ausbildung von Amyloid-Eiweißfasern (amyloid protein fibrils), vorzugsweise jene, welche als Amyloid-Plaques in Erscheinung treten und sich auf das Zentrale Nervensystem auswirken, im Zusammenhang stehen.

2. Verwendung von Verbindungen der Allgemeinen Formel II. 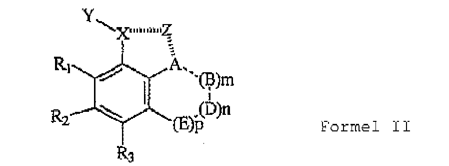 wobei:
X O, S darstellt;
Y H oder zusammen mit X, wo X = O ist, ein Kohlenhydratrest darstellt;
Z ein Metall- oder Seltenerdenkation darstellt, das radioaktiv sein kann oder nicht;
die l l l l l l l l Linie eine koordinative Bindung darstellt;
A N oder NR₄ darstellt;
B CR₅, NR₅ oder N darstellt ;
D CR₆, NR₆ oder N darstellt;
E CR₇, NR₇ oder N darstellt ;
mit der Bedingung, dass der Ring, welcher die Gruppe A enthält, ein in Maximum von zwei SLickstoffatomen in seiner Struktur aufweist;
m, n und p 0 oder 1 darstellen, wo m + n + p = 2 oder 3 ist;
die gestrichelten Linien - - - - eine Einfach- oder Doppelbindung darstellen;
R₁, R₂, R₃, R₄, R₅, R₆ und R₇ jeder unabhängig ein radioaktives Isotop, H, ein Halogen oder einen Rest optional mit einem radioaktiven Isotop darstellen, den Rest wählend aus: C₁-C₆ Alkyl, OH, C₁-C₆ Polyhydroxylalkyl, C₁-C₆ Alkoxyl, C₁-C₆ Alkoxylalkyl, (CH₂)q-OR' , wobei q 1, 2 oder 3 ist, CF₃, CH₂-CH₂F, O-CH₂-CH₂F, CH₂-CH₂-CH₂F, CN, NO₂, O(CO)R', OR', SR', COOR'-SO₃H, (CH₂) r-CO₂R", (CH₂)r-CO-R', wobei r 1, 2 oder 3 ist, und Rph, wobei Rph eine unsubstituierte oder substituierte Phenolgruppe darstellt, wobei die möglichen Substituenten der Phenolgruppe eine jede der Bedeutungen von R₁-R₇ mit Ausnahme einer Phenolgruppe sind;
R' H oder eine C₁₋₃ Alkylgruppe ist;
R" H, eine C₁-C₆ Alkylgruppe oder eine C₁-C₆ Alkoxylgruppe ist;
mit der Bedingung, dass nur einer von R₁, R₂, R₃, R₄, R₅, R₆, R₇, X, Y oder Z ein radioaktives Isotop ist oder aufweist;
in der Erstellung einer Zusammensetzung zur Diagnose und / oder Überwachung von Krankheiten, welche mit der Ausbildung von Amyloid-hiweißfasern (amyloid protein fibrils), vorzugsweise jene, welche als Amyloid-Plaques in Erscheinung treten und sich auf das Zentrale Nervensystem auswirken, im Zusammenhang stehen.

3. Verwendung von Verbindungen der Allgemeinen Formel III. 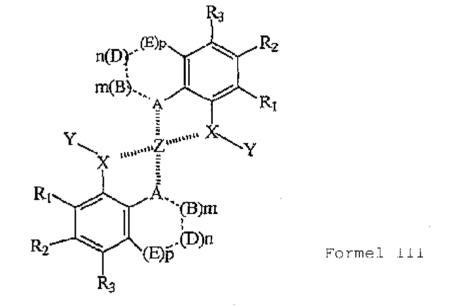 wobei:
X O, S darstellt;
Y H oder zusammen mit X, wo X = O ist, ein Kohlenhydratrest darstellt ;
Z ein Metall- oder Seltenerdenkation darstellt, das radioaktiv sein kann oder nicht;
die |||||||| Linie eine koordinative Bindung darstellt;
A N oder NR₄ darstellt ;
B CR₅, NR₅ oder N darstellt;
D CR₆, NR₆ oder N darstellt;
E CR₇, NR₇ oder N darstellt;
mit der Bedingung, dass der Ring, welcher die Gruppe A enthält, ein Maximum von zwei Stickstoffatomen in seiner Struktur aufweist;
m, n und p 0 oder 1 darstellen, wo m + n + p = 2 oder 3 ist;
die gestrichelten Linien - - - - eine Einfach- oder Doppelbindung darstellen;
R₁, R₂, R₃, R₄, R₅, R₆ und R₇ jeder unabhängig ein radioaktives Isotop, H, ein Halogen oder einen Rest optional mit einem radioaktiven Isotop darstellen, den Rost wählend aus: C₁-C₆ Alkyl, OH, C₁-C₆ Polyhydroxylalkyl, C₁-C₆ Alkoxyl, C₁-C₆ Alkoxylalkyl, (CH₂)_{q}-OR', wobei q 1, 2 oder 3 ist, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R", (CH₂)r-CO-R', wobei r 1, 2 oder 3 ist, und Rph, wobei Rph eine unsubstituierte oder substituierte Phenolgruppe darstellt, wobei die möglichen Substituenten der Phenolgruppe eine jede der Bedeutungen von R₁-R₇ mit Ausnahme einer Phenolgruppe sind;
R' H oder eine C₁₋₃ Alkylgruppe ist;
R" H, eine C₁-C₆ Alkylgruppe oder eine C₁-C₆ Alkoxylgruppe ist;
mit der Bedingung, dass nur einer von R₁, R₂, R₃, R₄, R₅, R₆, R₇, X, Y oder Z ein radioaktives Isotop ist oder aufweist ;
in der Erstellung einer Zusammensetzung zur Diagnose und / oder Überwachung von Krankheiten, welche miL der Ausbildung von Amyloid-Eiweißfasern (amyloid protein fibrils), vorzugsweise jene, welche als Amyloid-Plaques in Erscheinung treten und sich auf das Zentrale Nervensystem auswirken, im Zusammenhang stehen.

4. Verwendung nach Ansprüchen 1, 2 und 3 zur Diagnose und/oder Überwachung in Tieren, transgenen Tieren und insbesondere in Menschen von Krankheiten wie unter anderem Alzheimer, Parkinson, Huntington, zystische Fibrose, Altersdiabetes (late onset diabetes), AmyoLrophe Lateralsklerose, Mittelmeerfieber, Muckle-Wells Syndrom, idopathische Myelom, Amyloidpolyneuropathie, Amyloidkardiomyopathie, systemische senile Amyloidose, erbliche zerebrale Amyloid-Angiopathie, Down-Syndrom, Circulzfeldt-Jakob-Krankheit, Kuru, Gerstmann-Straussler-Schienker-Syndrom, medulläres Schilddrüsenkarzinome, Klappernamyloidablagerungen (amyloid valve deposits), Amyloidose bei Dialysepatienten, Einschlusskörpermyositis, Muskuläre Amyloidablagerungen, Sichelzellen-Parkinson-Anämie, Diabetes Typ 2.

5. Verbindungen von Allgemeinen Formel I 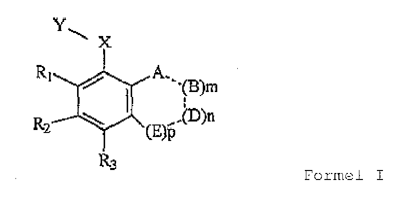 wobei:
X O, S darstellt;
Y H oder zusammen mit X, wo X = O ist, ein Kohlenhydratrest darstellt ;
A N oder NR₄ darstellt;
B CR₅, NR₅ oder N darstellt;
D CR₆, NR₆ oder N darstellt;
E CR₇, NR₇ oder N darstellt;
mit der Bedingung, dass der Ring, welcher die Gruppe A enthält, ein Maximum von zwei Stickstoffatomen in seiner Struktur aufweist;
m, n und p 0 oder 1 darstellen, wo m + n + p = 2 oder 3 ist;
die gestrichelten Linien - - - - eine Einfach- oder Doppelbindung darstellen;
R₁, R₂, R₃, R₄, R₅, R₆ und R₇ jeder unabhängig ein radioaktives Isotop, II, ein Halogen oder einen Rest optional mit einem radioaktiven Isotop darstellen, diesen Rest wählend aus: C₁-C₆ Alkyl, OH, C₁-C₆ Polyhydroxylalkyl, C₁-C₆ Alkoxyl, C₁-C₆ Alkoxylalkyl, (CH₂)q-OR', wobei q 1, 2 oder 3 ist, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R'', (CH₂)r-CO-R', wobei r 1, 2 oder 3 ist, und Rph, wobei Rph eine unsubstituierte oder substituierte Phenolgruppe darstellt, wobei die möglichen Substituenten der Phenolgruppe ein jede der Bedeutungen von R₁-R₇ mit Ausnahme einer Phenolgruppe sind;
R' H oder eine C₁₋₃ Alkylgruppe ist;
R'' H, eine C₁-C₆ Alkylgruppe oder eine C₁-C₆ Alkoxylgruppe ist;
mit der Bedingung, dass R₁, R₂, R₃, R₄, R₅, R₆, R₇, X und Y nicht alle gleichzeitig H sind, und mit der Bedingung, dass nur einer von R₁, R₂, R₃, R₄, R₅, R₆, R₇, X und Y ein radioaktives Isotop ist oder aufweist;

6. Verbindungen von Allgemeinen Formel II 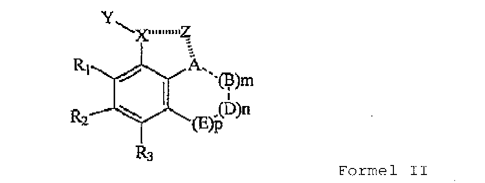 wobei:
X O, S darstellt;
Y H oder zusammen mit X, wo X = O ist, ein Kohlenhydratrest darstellt;
Z ein Metall- oder Seltenerden-Kation darstellt, das radioaktiv sein kann oder nicht ;
die |||||||| Linie eine koordinative Bindung darstellt;
A N oder NR₄ darstellt;
B CR₅, NR₅ oder N darstellt;
D CR₆, NR₆ oder N darstellt;
E CR₇, NR₇ oder N darstellt;
mit der Bedingung, dass der Ring, welcher die Gruppe A enthält, ein Maximum von zwei Stickstoffatomen in seiner Struktur aufweist;
m, n und p 0 oder 1 darstellen, wo m + n + p = 2 oder 3 ist;
die gestrichelten Linien - - - - eine Einfach- oder Doppelbindung darstellen;
R₁, R₂, R₃, R₄, R₅, R₆ und R₇ jeder unabhängig ein radioaktive Isotop, H, ein Halogen oder einen Rest optional mit einem radioaktiven Isotop darstellen, den Rest wählend aus: C₁-C₆ Alkyl, OH, C₁-C₆ Polyhydroxylalkyl, C₁-C₆ Alkoxyl, C₁-C₆ Alkoxylalkyl, (CH₂)q-OR', wobei q 1, 2 oder 3 ist, CF₃, CH₂CH₂F, O-CH₂-CH₂F, CH₂-CH₂-CH₂F, CN, NO₂, O(CO)R', OR', SR', COOR' - SO₃H, (CH₂)r-CO₂R'', (CH₂) r-CO-R', wobei r 1, 2 oder 3 ist, und Rph, wobei Rph eine unsubstituierte oder substituierte Phenolgruppe darstellt, wobei die möglichen Substituenten der Phenolgruppe ein jede der Bedeutungen von R₁-R₇ mit Ausnahme einer Phenolgruppe sind;
R' H oder eine C₁₋₃ Alkylgruppe ist;
R'' H, eine C₁-C₆ Alkylgruppe oder eine C₁-C₆ Alkoxylgruppe ist;
mit der Bedingung, dass R₁, R₂, R₃, R₄, R₅, R₆, R₇, X und Y nicht alle gleichzeitig H sind, und
mit der Bedingung, dass nur einer von R₁, R₂, R₃, R₄, R₅ R₆, R₇, X, Y oder Z ein radioaktives Isotop ist oder aufweist ;

7. Verbindungen von Allgemeinen Formel III 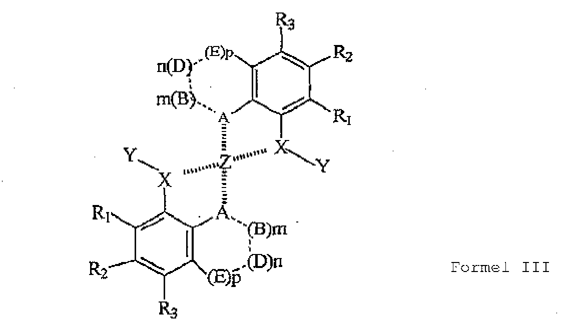 wobei:
X O, S darstellt;
Y H oder zusammen mit X, wo X = O, ein Kohlenhydratrest darstellt;
Z ein Metall- oder Seltenerdenkation darstellt, das radioaktiv sein kann oder nicht;
die | | | | | | | | Linie eine koordinative Bindung darstellt ;
A N oder NR₄ darstellt;
R CR₅, NR₅ oder N darstellt;
D CR₆, NR₆ oder N darstellt;
E CR₇, NF₇ oder N darstellt ;
mit der Bedingung, dass der Ring, welcher die Gruppe A enthält, ein Maximum von zwei Stickstoffatomen in seiner Struktur aufweist ;
m, n und p 0 oder 1 darstellen, wo m + n + p = 2 oder 3 ist ;
die gestrichelten Linien - - - - eine Einfach- oder Doppelbindung darstellen;
R₁, R₂, R₃, R₄, R₅, R₆, und R₇ jeder unabhängig ein radioaktives Isotop, H, ein Halogen oder einen Rest optional mit einem radioaktiven Isotop darstellen, den Rest wählend aus: C₁-C₆ Alkyl, OH, C₁-C₆ Polyhydroxylalkyl, C₁-C₆ Alkoxyl, C₁-C₆ Alkoxylalkyl, (CH₂)q-OR', wobei q 1, 2 oder 3 ist, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R'', (CH₂)r-CO-R', wobei r 1, 2 oder 3 ist , und Rph, wobei Rph eine unsubstituierte oder substituierte Phenolgruppe darstellt, wobei die möglichen Substituenten der Phenolgruppe ein jede der Bedeutungen von R₁-R₇ mit Ausnahme einer Phenolgruppe sind;
R' H oder eine C₁₋₃ Alkylgruppe ist;
R" H, eine C₁-C₆ Alkylgruppe oder eine C₁-C₆ Alkoxylgruppe ist;
mit der Bedingung, dass nur einer von R₁, R₂, R₃, R₄, R₅, R₆, R₇, X, Y oder Z ein radioaktives Isotop ist oder aufweist;
und mit der Bedingung dass, wenn
A N ist,
B, D und E alle CH sind,
X O ist, und
m, n und p alle 1 sind,
dann R₁, R₂ und R₃ nicht alle II sind.

8. Verbindungen nach Anspruch 5, **dadurch gekennzeichnet**, zu sein:
5-Chlor-7- [¹²⁴I] Iod-8-Hydroxychinolin
5-[¹²⁴I] Iod-8-Iod-8-Hydroxychinolin
5-Iod-7-[¹²⁴I]Iod-8-Hydroxychinolin
5-Chlor-7-[¹⁸F]Fluor-8-Hydroxychinolin
5- [¹⁸F]Fluor-7-Iod-8-Hydroxychinolin
5-Chlor-7-Iod-8-[¹¹C]Methoxychinolin
5-Chlor-7-[¹²⁴I]Iod-8-Hydroxychinolinglucuronid
5-Chlor-7-[¹²⁵I]Iod-8-Hydroxychinolinglucuronid
5-chlor-7-[¹³¹I] Iod-8-Hydroxychinolinglucuronid
5-Chlor-7- [¹⁸F] Fluor-8-Hydroxychinolinglucuronid
5- [¹⁸F] Fluor-7-Iod-8-Hydroxychinolinglucuronid
5-Chlor-7-Iod-8-[¹¹C]Methoxychinolinglucuronid
5- [¹²⁴T] Iod-8-Hydroxychinolin
7- [¹²⁴I] Iod-8-Hydroxychinolin
5-[¹⁸F]Fluor-8-Hydroxychinolin

9. Verbindungen nach Anspruch 5, **dadurch gekennzeichnet**, zu sein:
5-Chlor-7-[¹²³I]Iod-8-Hydroxychinolin
5-[¹²³I]Iod-7-Iod-8-Hydroxychinolin
5-Iod-7-[¹²³I]Iod-8-Hydroxychinolin
5-Chlor-7-[¹²³I]Iod-8-Hydroxychinolinglucuronid
5- [¹²³I] Iod-8-Hydroxychinolin
7-[¹²³I]Iod-8-Hydroxychinolin

10. Verbindungen nach Anspruch 6:
5-Chlor-7-[¹²⁴I]Iod-8-Hydroxychinolin-Fe(II)-Komplex
5-Chlor-7-[¹²⁴I]Iod-8-Hydroxychinolin-Cu(II)-Komplex
5-Chlor-7-[¹²⁴I]Iod-8-Hydroxychinolin-Zn(II)-Komplex
5-Chlor-7-[²⁴¹I]Iod-8-Hydroxychinolin-Mn(II)-Komplex
5-Chlor-7-[¹²⁵I]Iod-8-Hydroxychinolin-Fe(II)-Komplex
5-Chlor-7-[¹²⁵I]Iod-8-Hydroxychinolin-Cu(II)-Komplex
5-Chlor-7-[¹²⁵I]Iod-8-Hydroxynhinolin-Zn(II) -Komplex
5-Chlor-7-[¹²⁵I]Iod-8-Hydroxychinolin-Mn(II)-Komplex
5-Chlor-7-[¹⁸F]Fluor-8-Hydroxychinolin-Fe(II)-Komplex
5-Chlor-7-[¹⁸F]Fluor-8-Hydroxychinolin-Cu(II)-Komplex
5-Chlor-7-[¹⁸F]Fluor-8-Hydroxychinolin-Zn(II)-Komplex
5-Chlor-7-[¹⁸F]Fluor-8-Hydroxychinolin-Mn-(11)-Komplex
5- [¹⁸F] Fluor-7-Iod-8-Hydroxychinolin-Fe (II) -Komplex
5-[¹⁸F]Fluor-7-Iod-8-Hydroxychinolin-Cu(II)-Komplex
5- [¹⁸F]Fluor-7-Iod-8-Hydroxychinolin-Zn(II)-Komplex
5- [¹⁸F] Fluor-7-Iod-8-Hydroxychinolin-Mr (II) -Komplex
5-Chlor-7-Iod-8-[¹¹C]Methoxychinolin-Fc(II)-Komplex
5-Chlor-7-Iod-8-[¹¹C]Methoxychinolin-Cu(II)-Komplex
5-Chlor-7-Iod-8-[¹¹C]Methoxychinolin-Zn(II)-Komplex
5-Chlor-7-Iod-8-[¹¹C]Methoxychinolin-Mn(II)-Komplex

11. Verbindungen nach Anspruch 6:
5-Chlor-7-[¹²³I]Iod-8-Hydroxychinolin-Fe(II)-Komplex
5-Chlor-7- [¹²³I] Iod-8-Hydroxychinolin-Cu (II) -Komplex
5-Chlor-7-[¹²³I] Iod-8-Hydroxychinolin-Zn(II)-Komplex
5-Chlor-7-[¹²³I] Iod-8-Hydroxychinolin-Mn(II)-Komplex

12. Verbindungen nach Anspruch 6:
5-Chlor-7-Iod-8-Hydroxychinolin-^{99m}Tc-Komplex
5-Chlor-7-Iod-8-Hydroxychinolin-¹¹¹In-Komplex
5-Chlor-7-Iod-8-Hydroxychinolin-²⁰¹Tl-Komplex
5-Chlor-7-Iod-8-Hydroxychinolin-⁶⁷Ga-Komplex
5-Chlor-7-Iod-8-Hydroxychinolin-⁶⁸Ga-Komplex
5-Chlor-7-Iod-8-Hydroxychinolin-⁶⁷Cu-Komplex
5-Chlor-7-Iod-8-Hydroxychinolin-⁶⁴Cu-Komplex

13. Verbindungen nach Anspruch 7:
5-Chlor-7[¹²⁴T]Iod-8-Hydroxychinolin-Fe(II)-Bis-Chelat-Komplex
5-Chlor-7-[¹²⁴I]Iod-8-Hydroxychinolin-Cu(II)-Bis-Chelat-Komplex
5-Chlor-7-[¹²⁴I]Iod-8-Hydroxychinolin-Zn(II)-Bis-Chelat-Komplex
5-Chlor-7-[¹²⁴I]Iod-8-Hydroxychinolin-Mn(II)-Bis-Chelat-Komplex
5-Chlor-7-[¹⁸F]Fluor-8-Hydroxychinolin-Fe(11)-Bis-Chelat-Komplex
5-Chlor-7-[¹⁸F]Fluor-8-Hydroxychinolin-Cu(II)-Bis-Chelat-Komplex
5-Chlor-7-[¹⁸F]Fluor-8-Hydroxychinolin-Zn(II)-Bis-Chelat-Komplex
5-Chlor-7-[¹⁸F]Fluor-8-Hydroxychinolin-Mn(II)-Bis-Chelat-Komplex
5-[¹⁸F]Fluor-7-Iod-8-Hydroxychinolin-Fe(II)-Bis-Chelat-Komplex
5-[¹⁸F]Fluor-7-Iod-8-Hydroxychinolin-Cu(II)- Bis-Chelat-Komplex
5-[¹⁸F]Fluor-7-Iod-8-Hydroxychinolin-Zn(11)- Bis-Chelat-Komplex
5-[¹⁸F]Fluor-7-Iod-8-Hydroxychinolin-Mn(II)-Bis-Chelat-Komplex
5-Chlor-7-Iod-8-[¹¹C]Methoxychinolin-Fe(II)- Bis-Chelat-Komplex
5-Chlor-7-Iod-8-[¹¹C]Methoxychinolin-Cu(II)- Bis-Chelat-Komplex
5-Chlor-7-Iod-8-[¹¹C]Methoxychinolin-Zn(II)-Bis-Chelat-Komplex
5-Chlor-7-Iod-8-[¹¹C]Methoxychinolin-Mn(II)-Bis-Chelat-Komplex

14. Verbindungen nach Anspruch 7:
S-Chlor-7-[¹²³I]Iod-8-Hydroxychinolin-Fe(II)- Bis-Chelat-Komplex
5-Chlor-7-[¹²³I]Iod-8-Hydroxychinolin-Cu(II)-Bis-Chelat-Komplex
5-Chlor-7-[¹²³I]Iod-8-Hydroxychinolin-Zn(II)-Bis-Chelat-Komplex
5-Chlor-7- [¹²³I] Iod-8-Hydroxychinolin-Mn(II) - Bis-Chelat-Komplex

15. Verbindung nach Anspruch 7:
5-Chlor-7-Iod-8-Hydroxychinolin-^{99m}Tc-Bis-ChelatKomplex
5-Chlor-7-Iod-8-Hydroxychinolin-¹¹¹In-Bis-ChelatKomplex
5-Chlor-7-Iod-8-Hydroxychinolin-²⁰¹Tl-Bis-ChelatKomplex
5-Chlor-7-Iod-8-Hydroxychinolin-⁶⁷Ga-Bis-ChelatKomplex
5-Chlor-7-Iod-8-Hydroxychinolin-⁶⁸Ga-Bis-Chelat-Komplex
5-Chlor-7-Iod-8-Hydroxychinolin-⁶⁷Cu-Bis-ChelatKomplex
5-Chlor-7-Iod-8-Hydroxychinolin-⁶⁴Cu-Bis-ChelatKomplex

16. Pharmazeutische Zusammensetzung zur Diagnose von im Zusammenhang mit Eiweißablagerung im Zentralen Nervensystem stehenden Krankheiten mit einer der in den Ansprüchen 5 bis 12 definierten Verbindungen.

17. Verfahren zur Erstellung der in den Ansprüchen 5, 8 und 9 definierten Verbindungen mit:
a) Bewirken einer Reaktion eines Chinolinderivates mit einem elektophilen, aromatischen Halogenierungsreagenten, welcher ein radioaktives Halogenatom einschließt oder
b) Bewirken einer Reaktion eines Chinolinderivates mit einem radioaktiven, halogenierten Derivat, um ei ne aromatische, nukleophile Substitutionsreaktion zu bewirken.

18. Verfahren zur Erstellung der in den Ansprüchen 6, 10, 11 und 12 definierten Ansprüche mit:
a) Bewirken eine Reaktion eines Chinolinderivates mit einem Metall- oder Seltenerdenkation oder
b) Bewirken einer Reaktion eines Chinolinderivates mit einem radioaktivem Isotop dieser Elemente,
so dass das Metall- oder Seltenerdenkation oder das radioaktive Isotop dieser Elemente in einer geeigneten Oxidationsstufe ist, um so das in den Ansprüchen 6, 10, 11 und 12 definierte, entsprechende chelatbildende Produkt zu erstellen.

19. Verfahren zum Erstellen der in Anspruch 7 definierten Verbindungen mit Bewirken einer Reaktion eines Chelatderivates mit:
a) einem Metall- oder Seltenerdenkation, oder
b) einem radioaktiven Isotop dieser Elemente
in einer geeigneten Oxidationsstufe, um so das in den Ansprüchen 7, 13, 14 und 15 definierte, entsprechende chelatbildende Produkt zu erstellen.

20. Verfahren zur Bildgebung eines Gewebes des Zentralen Nervensystems eines Menschen, transgenen Tieres oder Tieres unter Verwendung einer Verbindung der Formeln I, II und III, wie in den Ansprüchen 1, 2 und 3 jeweils definiert oder einer Verbindung nach den Ansprüchen 8 bis 15.

21. Verfahren nach Anspruch 20, wobei das Gewebe durch Krankheiten befallen ist, welche mit der Bildung von Amyloid-Eiweißfasern, insbesondere solche, welche als Amyloid-Plaques in Erscheinung treten, im Zusammenhang stehen.

## Revendications

1. Utilisation d'un composé de formule générale I 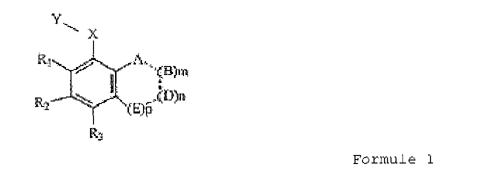 dans laquelle :
x représente O, S ;
Y représente H ou, avec X, où X = O, un radical glucidique ;
A représente N ou NR₄ ; 5
B représente CR₅, NR₅ ou N ;
D représente CR₆, NR₆ ou N ;
E représente CR₇, NR₇ ou N ;
à condition que le cycle contenant le groupe A ait un maximum de deux atomes d' azote dans sa structure ;
m, n et p représente 0 ou 1, où m + n + p = 2 ou 3;
les lignes pointillées - - - - représentent une liaison simple ou double ;
R₁, R₂, R₃, R₄, R₅, R₆ et R₇ représentent chacun indépendamment un isotope radioactif, H, un atome d'halogène ou un radical possédant facultativement un isotope radioactif, ledit radical étant choisi parmi les groupes alkyle en C₁-C₆, OH, polyhydroxyalkyle en C₁-C₆, alcoxyle en C₁-C₆, alcoxyalkyle en C₁-C₆, (CH₂)q-OR', où q vaut 1, 2 ou 3, CF₃, CH₂-CH₂F, O-CH₂-CH₂F, CH₂-CH₂-CH₂F, CN, NO₂, O(CO)R', OR', SR', COOR' -SO₃H, (CH₂)r-CO₂R", où r vaut 1, 2 ou 3 et Rph, où Rph représente un groupe phénol substitué ou non substitué, les substituants possibles du groupe phénol étant l'une quelconque des significations R₁-R₇ mis a part un groupe phénol ;
R' est H ou un groupe alkyle en C₁₋₃;
R" est H, un groupe alkyle en C₁-C₆ ou un groupe alkyloxy en C₁-C₆;
à condition que seulement un de R₁, R₂, R₃, R₄, R₅, R₆, R₇, X et Y soit ou possède un isotope radioactif ; pour la préparation d'une composition pour le diagnostic et/ou le contrôle de maladies associées à la formation de fibrilles amyloïdes de protéine, en particulier celles qui apparaissent sous forme de plaques amyloïdes et affectent le système nerveux central.

2. Utilisation des composés de formule générale II : 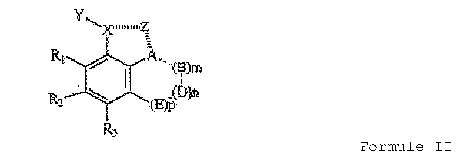 dans laquelle :
X représente O, S ;
Y représente H ou, avec X, où X - O, un radical glucidique ;
Z représente un métal ou un cation de terre rare peut ou ne pas être radioactif ;
la ligne | | | | | | | | représente une liaison de coordination ;
A représente N ou NR₄;
B représente CR₅, NR₅ ou N;
D représente CR₆, NR₆ ou N ;
E représente CR₇, NR₇ ou N ;
à condition que le cycle contenant le groupe A ait un maximum de deux atomes d'azote dans sa structure ;
m, n et p représentent 0 ou 1, où m + n + p = 2 ou 3;
les lignes pointillées - - - - représentent une liaison simple ou double ;
R₁, R₂, R₃, R₄, R₅, R₆ et R₇ représentent chacun indépendamment un isotope radioactif, H, un atome d'halogène ou un radical possédant facultativement un isotope radioactif, ledit radical étant choisi parmi les groupes alkyle en C₁-C₆, OH, polyhydroxyalkyle en C₁-C₆, alcoxyle en C₁-C₆, alcoxyalkyle en C₁-C₆, (CH₂)q-OR', où q vaut 1, 2 ou 3, CF₃, CH₂-CH₂F, O-CH₂-CH₂F, CH₂,-CH₂-CH₂F, CN, NO₂, O(CO)R', OR' , SR' , COOR' -SO₃H, (CH₂)r-CO₂R'', (CH₂)r-CO-R', où r vaut 1, 2 ou 3 et Rph, où Rph représente un groupe phénol substitué ou non substitué, les substituants possibles du groupe phénol étant l'une quelconque de significations R₁-R₇, mis à part un groupe phénol ;
R' esL H ou un groupe alkyle en C₁₋₃ ;
R" est II, un groupe alkyle en C₁-C₆ ou un groupe alkyloxy en C₁-C₆ ;
à condition que seulement un de R₁, R₂, R₃, R₄, R₅, R₆, R₇, X, Y ou Z soit ou possède un isotope radioactif ;
pour la préparation d'une composition pour le diagnostic et/ou le contrôle de maladies associées à la formation de fibrilles amyloïdes de protéine, en particulier celles qui apparaissent sous forme de plaques amyloïdes et affectent le système nerveux central.

3. Utilisation des composés de formule générale III : 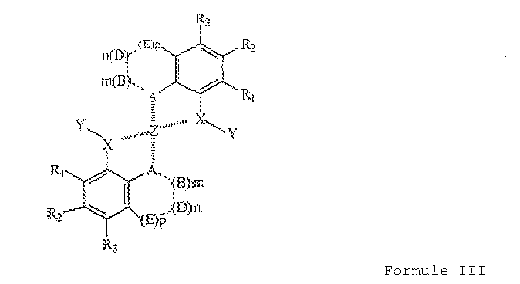 dans laquelle :
X représente O, S ;
Y représente H ou, avec X, où X - O, un radical glucidique ;
Z. représente un métal ou un cation de terre rare peut ou ne pas être radioactif ;
la ligne l l l l l l l l représente une liaison de coordination ;
A représente N ou NR₄ ;
B représente CR₅, NR₅ ou N ;
D représente CR₆, NR₆ ou N ;
E représente CR₇, NR₇ ou N ;
à condition que le cycle contenant le groupe A ait un maximum de deux atomes d'azote dans sa structure ;
m, n et p représentent 0 ou 1, où m + n + p = 2 ou 3 ;
les lignes pointillées - - - - représentent une liaison simple ou double ;
R₁, R₂, R₃, R₄, R₅, R₆ et R₇ représentent chacun indépendamment un isotope radioactif, II, un atome d'halogène ou un radical possédant facultativement un isotope radioactif, ledit radical étant choisi parmi les groupes alkyle en C₁-C₄, OH, polyhydroxyalkyle en C₁-C₆, alcoxyle en C₁-C₆, alcoxyalkyle en C₁-C₆, (CH₂)q-OR', où q vaut 1, 2 ou 3, CF₃, CH₂,-CH₂F, O-CH₂-CH₂F, CH₂-CH₂-CH₂F, CN, NO₂, O(CO)R', OR', SR', COOR' -SO₃H, (CH₂)r-C₂R", (CH₂) r-CO-R' , où r vaut 1, 2 ou 3 et Rph, où Rph représente un groupe phénol substitué ou non substitué, les substituants possibles du groupe phénol étant l'une quelconque des significations R₁-R₇ mis à part un groupe phénol ;
R' est H ou un groupe alkyle en C₁₋₃;
R" est H, un groupe alkyle en C₁-C₆ ou un groupe alkyloxy en C₁-C₆ ;
à condition que seulement un de R₁, R₂, R₃, R₄, R₅, R₆, R₇, X, Y ou Z soit ou possède un isotope radioactif ;
pour la préparation d'une composition pour le diagnostic et/ou le contrôle de maladies associées à la formation de fibrilles amyloïdes de protéine, en particulier celles qui apparaissent sous forme de plaques amyloïdes et affectent le système nerveux central.

4. Utilisation selon les revendications 1, 2 et 3 pour le diagnostic et/ou le contrôle chez des animaux, des animaux transgéniques, et en particulier des être humains, de maladies telles que la maladie d'Alzheimer, la maladie de Parkinson, la maladie d'Huntington, la mucoviscidose, le diabète tardif, la sclérose latérale amyotrophique, la brucellose, le syndrome de Muckle-Wells, le myélome idiopathique, le polyneuropathie amyloïde, la cardiomyopathie amyloïde, l'amyloïdose systémique sénile, l'hémorragie cérébrale héréditaire avec amyloïdose, la trisomie 21, la maladie de Creutzfeld-Jacob, le kuru, le syndrome de Cerstmann-Straussler-Schienker, le cancer médullaire de la thyroïde, le dépôt amyloïde au niveau valvulaire, l'amyloïdose chez des patients sous dialyse, la myosile à corps d'inclusion, les dépôts amyloïdes au niveau musculaire, la dépanocytose de Parkinson, le diabète de type 2, entre autres.

5. Composés de formule générale 1 : 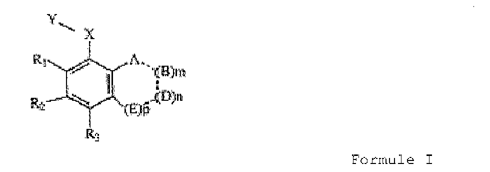 dans laquelle :
X représente O, S ;
Y représente H ou, avec X, où X = O, un radical glucidique ;
A représente N ou NR₄ ;
B représente CR₅, NR₅ ou N ;
D représente CR₆, NR₆ ou N ;
E représente CR₇, NR₇ ou N ;
à condition que le cycle contenant le groupe A ait un maximum de deux atomes d'azote dans sa structure ;
m, n et p représentent 0 ou 1, où m + n + p = 2 ou 3;
les lignes pointillées - - - - représentent une liaison simple ou double ;
R₁, R₂, R₃, R₄, R₅, R₆ et R₇ représentent chacun indépendamment un isotope radioactif, H, un atome d'halogène ou un radical possédant facultativement un isotope radioactif, ledit radical étant choisi parmi les groupes alkyle en C₁-C₆, OH, polyhydroxyalkyle en C₁-C₆, alcoxyle en C₁-C₆, alcoxyalkyle en C₂-C₆, (CH₂)q-OR' , où q vaut 1, 2 ou 3, CF₃, CH₂-CH₂F, O-CH₂-CH₂F, CH₂-CH₂-CH₂F, CN, NO₂, O(CO)R', OR', SR' , COOR' -SO₃H, (CH₂)r-CO-R'', (CH₂)r-CO-R', où r vaut 1, 2 ou 3 et Rph, où Rph représente un groupe phénol substitué ou non substitué, les substituants possibles du groupe phénol étant l'une quelconque des significations R₁-R₇ mis à part un groupe phénol ;
R' est H ou un groupe alkyle en C₁₋₃ ;
R'' est H, un groupe alkyle en C₁-C₆ ou un groupe alkyloxy en C₁-C₆ ;
à condition que R₁, R₂, R₃, R₄, R₅, R₆, R₇, X et Y ne soient pas simultanément H, et
à condition que seulement un de R₁, R₂, R₃, R₄, R₅, R₆, R₇, X ou Y soit ou possède un isotope radioactif.

6. Composés de formule générale II : 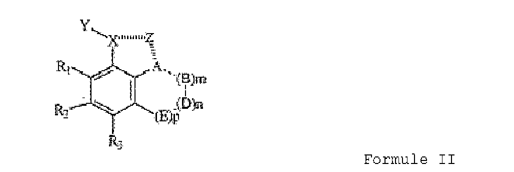 dans laquelle :
X représente O, S ;
Y représente H ou, avec X, où X = O, un radical glucidique ;
Z représenta un métal ou un cation de terre rare peut ou ne pas être radioactif ;
la ligne l l l l l l l l représente une liaison de coordination;
A représente N ou NR₄ ;
B représente CR₅, NR₅ ou N ;
D représente CR₆, NR₆ ou N ;
E représente CR₇, NR₇, ou N ;
à condition que le cycle contenant le groupe A ait un maximum de deux atomes d'azote dans sa structure ;
m, n et p représentent 0 ou 1, où m + n + p = 2 ou 3;
les lignes pointillées - - - - représentent une liaison simple ou double ;
R₁, R₂, R₃, R₄, R₅, R₆, et R₇ représentent chacun indépendamment un isotope radioactif, H, un atome d'halogène ou un radical possédant facultativement un isotope radioactif, ledit radical étant choisi parmi les groupes alkyle en C₁-C₆, OH, polyhydroxyalkyle en C₁-C₆, alcoxyle en C₁-C₆, alcoxyalkyle en C₁-C₆, (CH₂)q-OR'**,** où q vaut 1, 2 ou 3, CF₃, CH₂-CH₂F, O-CH₂-CH₂F, CH₂CH₂-CH₂F, CN, NO₂, O(CO)R', OR', SR', COOR' -SO₃H, (CH₂) r-CO₂R", (CH₂)r-CO-R', où r vaut 1, 2 ou 3 et Rph, où Rph représente un groupe phénol substitué ou non substitué, les substituants possibles du groupe phénol étant l'une quelconque des significations R₁-R₇ mis à part un groupe phénol ;
R' est H ou un groupe alkyle en C₁₋₃;
R" est H, un groupe alkyle en C₁-C₆ ou un groupe alkyloxy en C₁-C₆ ;
à condition que R₁, R₂, R₃, R₄, R₅, R₆, R₇, X et Y ne soient pas simultanément H, et
à condition que seulement un de R₁, R₂, R₃, R₄, R₅, R₆, R₇, X, Y ou Z soit ou possède un isotope radioactif.

7. Composés de formule générale III : 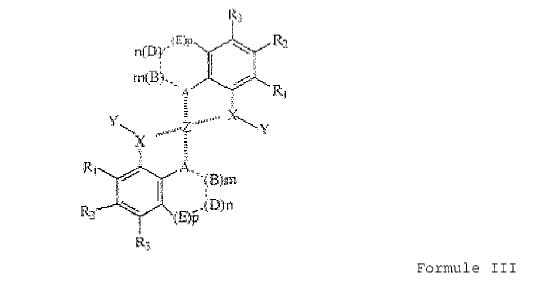 dans laquelle :
X représente O, S ;
Y représente H ou, avec X, où X - O, un radical glucidique ;
Z représente un métal ou un cation de terre rare peut ou ne pas être radioactif ;
la ligne |||||||| représente une liaison de coordination ;
A représente N ou NR₄ ;
B représente CR₅, NR₅, ou N ;
D représente CR₆, NR₆ ou N ;
E représente CR₇, NR₇ ou N ;
à condition que le cycle contenant le groupe A ait un maximum de deux atomes d'azote dans sa structure ;
m, n et p représentent 0 ou 1, où m + n + p = 2 ou 3;
les lignes pointillées - - - - représentent une liaison simple ou double ;
R₁, R₂, R₃, R₄, R₅, R₆ et R₇, représentent chacun indépendamment un isotope radioactif, H, un atome d'halogène ou un radical possédant facultativement un isotope radioactif, ledit radical étant choisi parmi les groupes alkyle en C₁-C₆, OH, polyhydroxyalkyle en C₁-C₆, alcoxyle en C₁-C₆, alcoxyalkyle en C₁-C₆, (CH₂) q-OR' , où q vaut 1, 2 ou 3, CF₃, CH₂-CH₂F, O-CH₂-CH₂F, CH₂-CH₂-CH₂F', CN, NO-, O(CO)R', OR' , SR', COOR' -SO₃H, (CH₂)r-CO-R" (CH₂) r-CO-R' , où r vaut 1, 2 ou 3 et Rph, où Rph représente un groupe phénol substitué ou non substitué, les substituants possibles du groupe phénol étant l'une quelconque des significations R₁-R₇ mis a part un groupe phénol ;
R' est H ou un groupe alkyle en C₁₋₃ ;
R" est H, un groupe alkyle en C₁-C₆ ou un groupe: alkyloxy en C₁-C₆;
à condition que seulement un de R₁, R₂, R₃, R₄, R₅, R₆, R₇, X, Y ou Z soit ou possède un isotope radiocatif; et à condition que lorsque
A est N,
B, D et E sont tous CH,
X est O, et
m, n et p valent tous 1,
alors R₁, R₂ et R₃ ne soient pas tous H.

8. Composés selon la revendication 5, **caractérisés** comme étant :
5-chloro-7-[¹²⁴I]iodo-8-hydroxyquinoléine
5-[¹²⁴I]iodo-7-iodo-8-hydroxyquinoléine
5-iodo-7-[¹²⁴I]iodo-8-hydroxyquinoléine
5-chloro-7-[¹⁸F]fluoro-8-hydroxyquinoléine
5-[¹⁸F]fluoro-7-iodo-8-hydroxyquinoléine
5-chloro-7-iodo-8-[¹¹C]méthoxyquinoléine
5-chloro-7-[¹²⁴I]iodo-8-hydroxyquinoléine glucuronide
5-chloro-7-[¹²⁵I]iodo-8-hydroxyquinoléine glucuronide
5-chloro-7-[¹³¹I]iodo-8-hydroxyquinoléine glucuronide
5-chloro-7-[¹⁸F]fluoro-8-hydroxyquinoléine glucuronide
5-[¹⁸F] fluoro-7-iodo-8-hydroxyquinoléine glucuronide
5-chloro-7-iodo-8-[¹¹C]méthoxyquinoléine glucuronide
5-[¹²⁴I]-8-hydroxyquinoléine
7-[¹²⁴I] -8-hydroxyquinoléine
5-[¹⁸F] -8-hydroxyquinoléine

9. Composés selon la revendication 5, **caractérisés** comme étant :
5-chloro-7-[¹²³I]iodo-8-hydroxyquinoléine
5-[¹²³I]iodo-7-iodo-8-hydroxyquinoléine
5-iodo-7-[¹²³I]iodo-8-hydroxyquinoléine
5-chloro-7-[¹²³I]iodo-8-hydroxyquinoléine glucuronide
5-[¹²³I]-8-hydroxyquinoléine
7-[¹²³I]-8-hydroxyquinoléine

10. Compounds according to claim 6:
Complexe Fe (II) de 5-chloro-7-[¹²⁴I]iodo-8-hydroxyquinoléine
Complexe Cu(II) de 5-chloro-7-[¹²⁴I]iodo-8-hydroxyquinoléine
Complexe Zn (II) de 5-chloro-7-[¹²⁴I]iodo-8-hydroxyquinoléine
Complexe Mn(II) de 5-chloro-7-[¹²⁴I]iodo-8-hydroxyquinoléine
Complexe Fe(II) de 5-chloro-7-[¹²⁵I]iodo-8-hydroxyquinoléine
Complexe Cu(II) de 5-chloro-7-[¹²⁵I]iodo-8-hydroxyquinoléine
Complexe Zn (II) de 5-chloro-7-[¹²⁵I]iodo-8-hydroxyquinoléine
Complexe Mn(II) de 5-chloro-7-[¹²⁵I]iodo-8-hydroxyquinoléine
Complexe Fe(II) de 5-chloro-7-[¹⁸F]fluoro-8-hydroxyquinoléine
Complexe Cu(II) de 5-chloro-7-[¹⁸F]fluoro-8-hydxoxyquinoléine
Complexe Zn (II) de 5-chloro-7-[¹⁸F] fluoro-8-hydroxyquinoléine
Complexe Mn(II) de 5-chloro-7-[¹⁸F]fluoro-8-hydroxyquinoléine
Complexe Fe (II) de 5-[¹⁸F]fluoro-7-iodo-8-hydroxyquinoléine
Complexe Cu (II) de 5-[¹⁸F]fluoro-7-iodo-8-hydroxyquinoléine
Complexe Zn(II) de 5- [¹⁸F] fluoro-7-iodo-8-hydroxyquinoléine
Complexe Mn(II) de 5-[¹⁸F] fluoro-7-iodo-8-hydroxyquinoléine
Complexe Fe(II) de 5-chloro-7-iodo-8-[¹¹C]méthoxyquinoléine
Complexe Cu (II) de 5-chloro-7-iodo-8- [¹¹C] méthoxyquinoléine
Complexe Zn (II) de 5 chloro-7-iodo-8-[¹¹C]méthoxyquinoléine
Complexe Mn(II) de 5-chloro-7-iodo-8-[¹¹C]méthoxyquinoléine

11. Composé selon la revendication 6 :
Complexe Fc(II) de 5-chloro-7-[¹²³I]iodo-8-hydroxyquinoléine
Complexe Cu(II) de 5-chloro-7-[¹²³I]iodo-8-hydroxyquinoléine
Complexe Zn (II) de 5-chloro-7-[¹²³I]iodo-8-hydroxyquinoléine
Complexe Mn (II) de 5-chloro-7-[¹²³I]iodo-8-hydroxyquinoléine

12. Composé selon la revendication 6 :
Complexe ^{99m}Tc de 5-chloro-7-iodo-8-hydroxyquinoléine
Complexe ¹¹¹In de 5 chloro-7-iodo-8-hydroxyquinoléine
Complexe ²⁰¹Tl de 5-chloro-7-iodo-8-hydroxyquinoléine
Complexe ⁶⁷Ga de 5-chloro-7-iodo-8-hydroxyquinoléine
Complexe ⁶⁸Ca de 5-chloro-7-iodo-8-hydroxyquinoléine
Complexe ⁶⁷Cu de 5-chloro-7-iodo 8-hydroxyquinoléine
Complexe ⁶⁴Cu de 5-chloro-7-iodo-8-hydroxyquinoléine

13. Composé selon la revendication 7 :
Complexe bis-chélaté Fe(II) de 5-chloro-7-[¹²⁴I]iodo-8-hydroxyquinoléine
Complexe bis-chélaté Cu (II) de 5-chloro-7-[¹²⁴I] iodo-8-hydroxyquinoléine
Complexe bis-chélaté Zn (II) de 5-chloro-7- [¹²⁴I]iodo-8-hydroxyquinoléine
Complexe bis-chélaté Mn(II) de 5-chloro-7-[¹²⁴I]iodo-8-hydroxyquinoléine
Complexe bis-chélaté Fe(II) de 5-chloro-7-[¹⁸F]fluoro-8-hydroxyquinoléine
Complexe bis-chélaté Cu(II) de 5-chloro-7-[¹⁸F]fluoro-8-hydroxyquinoléine
Complexe bis-chélaté Zn(II) de 5-chloro-7-[¹⁸F]fluoro-8-hydroxyquinoléine
Complexe bis-chélaté Mn(TT) de 5-chloro-7-[¹⁸F]fluoro-8-hydroxyquinoléine
Complexe bis-chélaté Fe(II) de 5-[¹⁸F] fluoro-7-iodo-8-hydroxyquinoléine
Complexe bis-chélaté Cu(II) de 5-[¹⁸F] fluoro-7-iodo-8-hydroxyquinoléine
Complexe bis-chélaté Zn(II) de 5-[¹⁸F] fluoro-7-iodo-8-hydroxyquinoléine
Complexe bis-chélaté Mn (II) de 5-[¹⁸F]fluoro-7-iodo-8-hydroxyquinoléine
Complexe bis-chélaté Fe (II) de 5-chloro-7-iodo-8-[¹¹C]méthoxyquinoléine
Complexe bis-chélaté Cu (II) de 5-chloro-7-iodo-8-[¹¹C]méthoxyquinoléine
Complexe bis-chélaté Zn(II) de 5-chloro-7-iodo-8-[¹¹C]méthoxyquinoléine
Complexe bis-chélaté Mn(II) de 5-chloro-7-iodo-8-[¹¹C]méthoxyquinoléine

14. Compounds according to claim 7:
Complexe bis-chélaté Fe (II) de 5-chloro-7-[¹²³I]iodo-8-hydroxyquinoléine
Complexe bis-chélaté Cu(II) de 5-chloro-7-[¹²³I]iodo-8-hydroxyquinoléine
Complexe bis-chélaté Zn(II) de 5-chloro-7-[¹²³I]iodo-8-hydroxyquinoléine
Complexe bis-chélaté Mn(II) de 5-chloro-7-[¹²³I]iodo-8-hydroxyquinoléine Mn(II)

15. Compound according to claim 7:
Complexe bis-chélaté ^{99m}Tc de 5-chloro-7-iodo-8-hydroxyquinoléine
Complexe bis-chélaté ¹¹¹In de 5-chloro-7-iodo-8-hydroxyquinoléine
Complexe bis-chélaté ²⁰¹Tl de 5-chloro-7-iodo-8-hydroxyquinoléine
Complexe bis-chélaté ⁶⁷Ga de 5-chloro-7-iodo-8-hydroxyquinoléine
Complexe bis-chélaté ⁶⁸Ga de 5-chloro-7-iodo-8-hydroxyquinoléine
Complexe bis-chélaté ⁶⁷Cu de 5-chloro-7-iodo-8-hydroxyquinoléine
Complexe bis-chélaté ⁶⁴Cu de 5-chloro-7-iodo-8-hydroxyquinoléine

16. Composition pharmaceutique pour le diagnostic de maladies associées au dépôt de protéine dans le système nerveux central, comprenant l'un des composés définis selon les revendications 5 à 12.

17. Procédé pour la préparation des composés définis selon les revendications 5, 8 et 9, comprenant les étapes consistant à :
a) faire réagir un dérivé de la quinoléine avec un réactif d'halogénation aromatique électrophile incorporant un atome d'halogène radioactif, ou
b) faire réagir un dérivé de la quinoléine avec un dérivé halogéné radioactif pour réaliser une réaction de substitution nucléophile aromatique.

18. Procédé pour la préparation des composes définis selon les revendications 6, 10, 11 et 12, comprenant les étapes consistant à :
a) faire réagir un dérivé de la quinoléine avec un métal ou un cation de terre rare, ou,
b) faire réagir un dériva de la quinoléine avec un isotope radioactif de ces éléments,
de sorte que le métal ou le cation de terre rare ou l'isotope radioactif de ces éléments soit dans un état d'oxydation approprié afin de produire le produit chélaté correspondant défini selon les revendications 6, 10, 11 et 12.

19. Procédé pour la préparation des composés définis selon la revendication 7, comprenant la préparation d'un dérivé de la quinoléine avec :
a) un métal ou un cation de terre rare, ou,
b) un isotope radioactif de ces éléments, dans un état d'oxydation approprié afin de produire le produit chélaté correspondant défini selon les revendications 7, 13, 14 et 15.

20. Procédé d'imagerie d'un tissu du système nerveux central d'un être humain, d'un animal transgénique ou d'un animal, en utilisant un composé des formules I, II et III. comme définies selon les revendications 1, 2 et 3 respectivement ou un composé selon les revendications 8 à 15.

21. Procédé selon la revendication 20, dans lequel le tissu est affecté par des maladies associées à la formation de fibrilles amyloïdes de protéine, en particulier celles qui apparaissent sous la forme de plaques amyloïdes.
